# EUROPEAN PATENT APPLICATION

(11) **EP 1 962 083 A1**
(43) Date of publication of application: **27.08.2008**
(21) Application number: 08003294.9
(22) Date of filing: 22.02.2008
(51) Int. Cl.: G01N 21/47, A61B 3/12, G01B 9/02, G01B 11/06

(54) **Optical image measurement device**

(30) Priority: 23.02.2007 JP 2007044653
(71) Applicant: Kabushiki Kaisha TOPCON, Tokyo (JP)
(72) Inventor: Kikawa, Tsutomu, Tokyo (JP); Suwa, Akihiro, Tokyo (JP)
(74) Representative: Gagel, Roland

(57) **Abstract**

An optical image measurement device comprises: a controller configured to control the scanner to scan the target position along a plurality of radially-arranged scanning lines; and an image forming part configured to form a tomographic image on each of the plurality of scanning lines based on the result detected by the detector, and form a 3-dimensional image of the measurement object based on the plurality of tomographic images having been formed.

## Description

### BACKGROUND OF THE INVENTION

### Field of the Invention

The present invention relates to an optical image measurement device configured to apply a light beam to a measurement object and form an image of the surface morphology or internal morphology of the measurement object by using a reflected light or a transmitted light.

### 2. Description of the Related Art

In recent years, attention has been focused on an optical image measurement technology of forming an image showing the surface morphology or internal morphology of a measurement object by using a light beam from a laser light source or the like. Because this optical image measurement technology does not have invasiveness to human bodies unlike an X-ray CT device, it is particularly expected to use this technology in the medical field.

Japanese Unexamined Patent Application Publication JP-A 11-325849 discloses an optical image measurement device having a configuration that: a measuring arm scans an object through a rotary deflection mirror (Galvano mirror); a reference mirror is disposed to a reference arm; an interferometer is used at the outlet so that the intensity of light appearing from interference of light fluxes from the measuring arm and the reference arm is analyzed by a spectrometer; and a device gradually changing the light flux phase of the reference light in non-continuous values is disposed to the reference arm.

The optical image measurement device of JP-A 11-325849 uses a method of so-called "Fourier Domain Optical Coherence Tomography (OCT)" based on technology of German Patent Application Publication DE4309056A1. That is to say, a beam of a low-coherence light is applied to a measurement object, the spectrum intensity distribution of a reflected light is obtained, and the obtained distribution is subjected to Fourier conversion, whereby an image of the morphology of the measurement object in a depth direction (z-direction) is formed.

Furthermore, the optical image measurement device described in JP-A 11-325849 is provided with a Galvano mirror that scans with an optical beam (signal light), whereby it is possible to form an image of a desired measurement region of a measurement object. Because this optical image measurement device is configured to scan with a light beam only in one direction (x-direction) orthogonal to the z-direction, a formed image is a 2-dimensional cross-sectional image of the depth direction (z-direction) along the light beam scanning direction (x-direction).

Furthermore, Japanese Unexamined Patent Application Publication JP-A 2002-139421 discloses a technique of scanning with a signal light in the horizontal and vertical directions to form a plurality of 2-dimensional tomographic images in the horizontal direction and, based on the plurality of tomographic images, acquiring 3-dimensional tomographic information of a measurement range to image. As this 3-dimensional imaging, for example, a method of displaying a plurality of tomographic images side by side in the vertical direction (referred to as stack data or the like), a method of forming a 3-dimensional image by applying a rendering process to a plurality of tomographic images, and the like are conceivable.

Besides, Japanese Unexamined Patent Application Publication JP-A 2003-543 discloses a configuration in which the aforementioned optical image measurement device is applied to the ophthalmology field.

With the conventional optical image measurement devices as described above, it is required to perform measurement along a number of scanning lines in order to improve the accuracy of a 3-dimensional image (including stack data) of a measurement object, whereby the measurement is prolonged.

If the measurement takes a long time as described above, a problem may occur at the time of measurement of a measurement object such as a living body. For example, in the case of measurement of a fundus oculi in a living body, the accuracy of a 3-dimensional image may be decreased by effects of small involuntary eye movement or eye movement due to a blood flow or the like. For example, with the configuration described in JP-A 2002-139421, relative positions of a plurality of tomographic images may be misaligned due to eye movement during measurement, whereby the positional accuracy of a 3-dimensional image may be decreased. Therefore, measurements such as shortening the measurement time have been taken by reducing the number of scanning lines or narrowing a measurement region.

However, reduction of the number of scanning lines decreases the accuracy of a 3-dimensional image, and is hard to regard as a favorable measure. Moreover, when a measurement region is narrowed, such a problem will occur that a 3-dimensional image of the whole site of a diagnostic object cannot be obtained.

Further, in measurement with an optical image measurement device, a low-coherence light having a center wavelength in the near-infrared region is often used. This low-coherence light also includes visible light components. Therefore, at the time of scan of a fundus oculi with a signal light, there arises a problem that a subject follows a scanning trajectory with eyes and is unable to fix the eyes. In particular, since a linear image moving in the vertical direction is viewed at the time of scan with a signal light as described in JP-A 2002-139421, the viewpoint of the eye often moves in the vertical direction.

The present invention has been devised to solve the abovementioned problems, and an object of the present invention is to provide an optical image measurement device capable of effectively conducting fixation when acquiring an image of an eye.

Further, another object of the present invention is to provide an optical image measurement device capable of acquiring a high-quality image while shortening a measurement time.

### SUMMARY OF THE INVENTION

In order to achieve the above objects, in a first aspect of the present invention, an optical image measurement device has: a light source configured to emit a low-coherence light; an interference-light generator configured to generate an interference light by dividing the emitted low-coherence light into a signal light and a reference light and superimposing the signal light having passed through a measurement object on the reference light; a scanner configured to scan a target position of the signal light on the measurement object; and a detector configured to detect the generated interference light, and the optical image measurement device forms an image of the measurement object based on a result detected by the detector, and the optical image measurement device comprises: a controller configured to control the scanner to scan the target position along a plurality of radially-arranged scanning lines; and an image forming part configured to form a tomographic image on each of the plurality of scanning lines based on the result detected by the detector and form a 3-dimensional image of the measurement object based on the plurality of tomographic images having been formed.

In a second aspect of the present invention, an optical image measurement device has: a light source configured to emit a low-coherence light; an interference-light generator configured to generate an interference light by dividing the emitted low-coherence light into a signal light and a reference light and superimposing the signal light passed through a measurement object onto the reference light; a scanner configured to scan a target position of the signal light on the measurement object; and a detector configured to detect the generated interference light, and the optical image measurement device forms an image of the measurement object based on a result detected by the detector, and the optical image measurement device comprises: a controller configured to control the scanner to scan the target position along a plurality of radially-arranged scanning lines; an image forming part configured to form a tomographic image on each of the plurality of scanning lines based on the result detected by the detector; and an analyzer configured to analyze the plurality of formed tomographic images to generate characteristic information representing characteristics of the measurement object.

### BRIEF DESCRIPTION OF THE DRAWINGS

Fig. 1 is a schematic configuration diagram showing an example of the entire configuration in a preferred embodiment of a device according to the present invention.
Fig. 2 is a schematic configuration diagram showing an example of the configuration of a scan unit installed in a retinal camera unit in the preferred embodiment of the device according to the present invention.
Fig. 3 is a schematic configuration diagram showing an example of the configuration of an OCT unit in the preferred embodiment of the device according to the present invention.
Fig. 4 is a schematic block diagram showing an example of the hardware configuration of an arithmetic control unit in the preferred embodiment of the device according to the present invention.
Fig. 5 is a schematic block diagram showing an example of the configuration of a control system in the preferred embodiment of the device according to the present invention.
Fig. 6 is a schematic view showing an example of the appearance of an operation panel in the preferred embodiment of the device according to the present invention.
Fig. 7 is a schematic view showing an example of a scanning pattern of a signal light in the preferred embodiment of the device according to the present invention.
Fig. 8 is a schematic view showing an example of a scanning pattern of a signal light in the preferred embodiment of the device according to the present invention.
Fig. 9 is a flowchart showing an example of a usage pattern in the preferred embodiment of the device according to the present invention.
Fig. 10 is a schematic view showing an example of a display screen in a usage pattern in the preferred embodiment of the device according to the present invention.
Fig. 11 is a schematic view showing an example of a display screen in a usage pattern in the preferred embodiment of the device according to the present invention.
Fig. 12 is a schematic view showing an example of a display screen in a usage pattern in the preferred embodiment of the device according to the present invention.
Fig. 13 is a schematic view showing an example of a display screen in a usage pattern in the preferred embodiment of the device according to the present invention.
Fig. 14 is a flowchart showing an example of a usage pattern in the preferred embodiment of the device according to the present invention.
Fig. 15 is a flowchart showing an example of a usage pattern in the preferred embodiment of the device according to the present invention.
Fig. 16 is a schematic block diagram showing an example of the configuration of the control system in the preferred embodiment of the device according to the present invention.
Fig. 17 is a schematic view showing an example of characteristic information generated by the preferred embodiment of the device according to the present invention.

### DETAILED DESCRIPTION OF THE PREFERRED EMBODIMENT

An example of a preferred embodiment of an optical image measurement device according to the present invention will be described in detail referring to the drawings.

Hereinafter, two embodiments related to the present invention will be described. A first embodiment shows formation of a 3-dimensional image based on a plurality of tomographic images of a measurement object. Further, a second embodiment shows generation of predetermined characteristic information based on a plurality of tomographic images of a measurement object.

### <First Embodiment>

### [Configuration of Device]

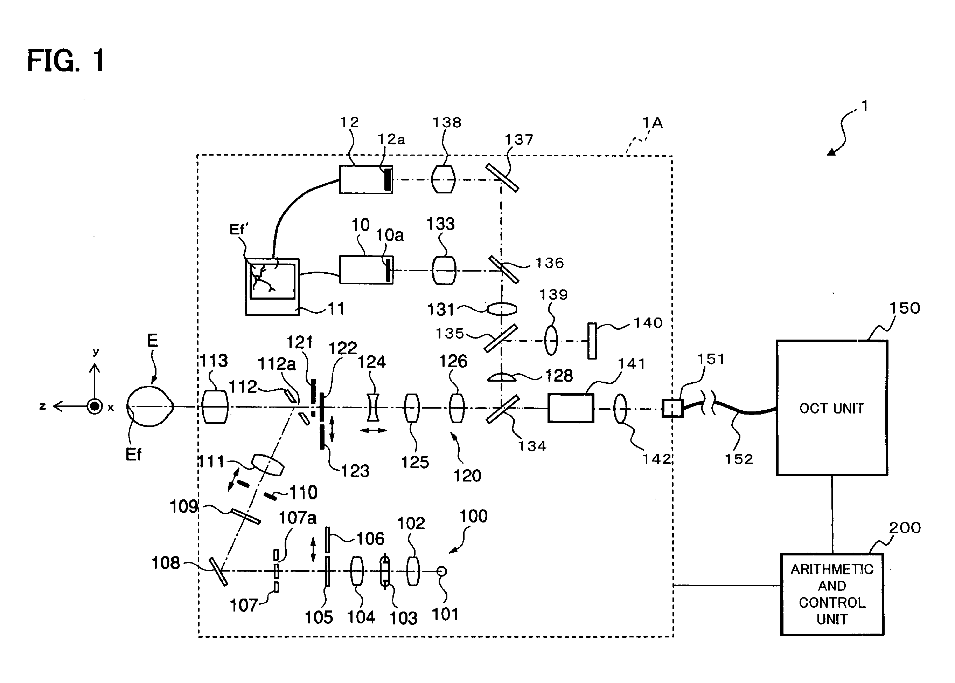

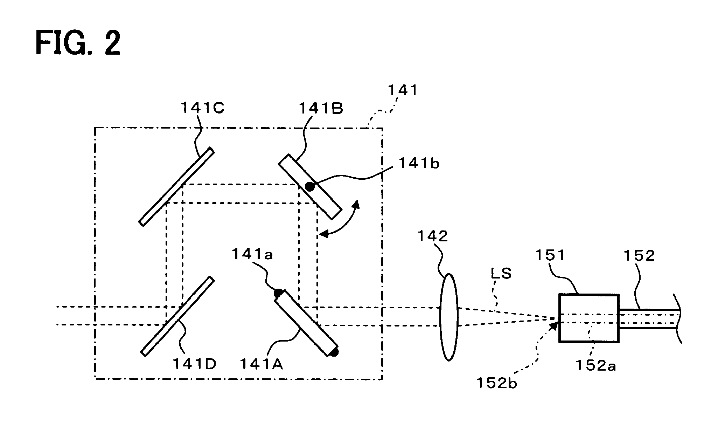

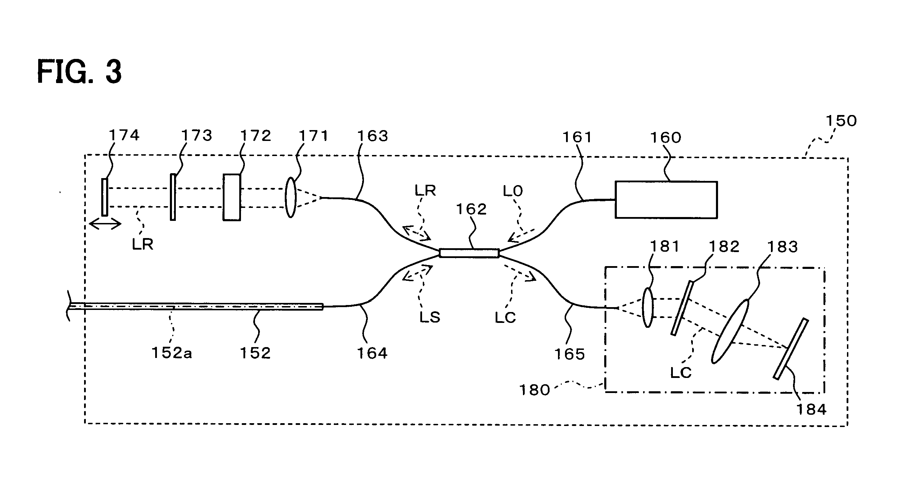

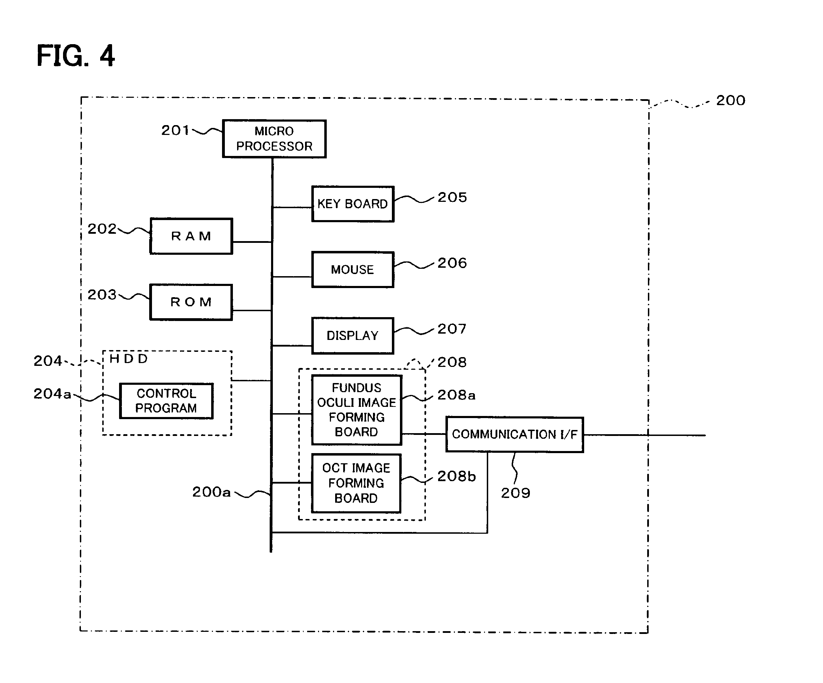

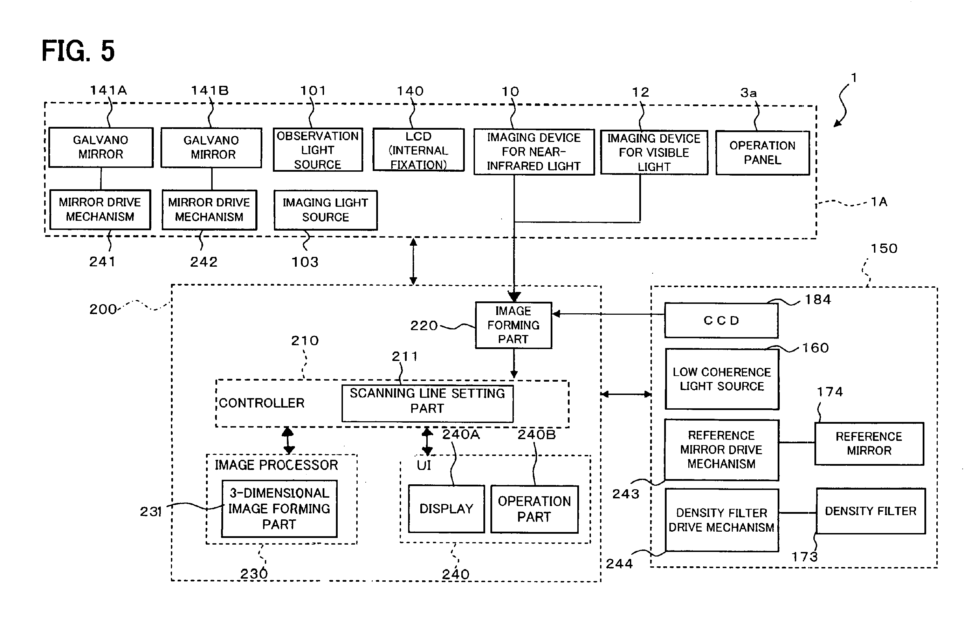

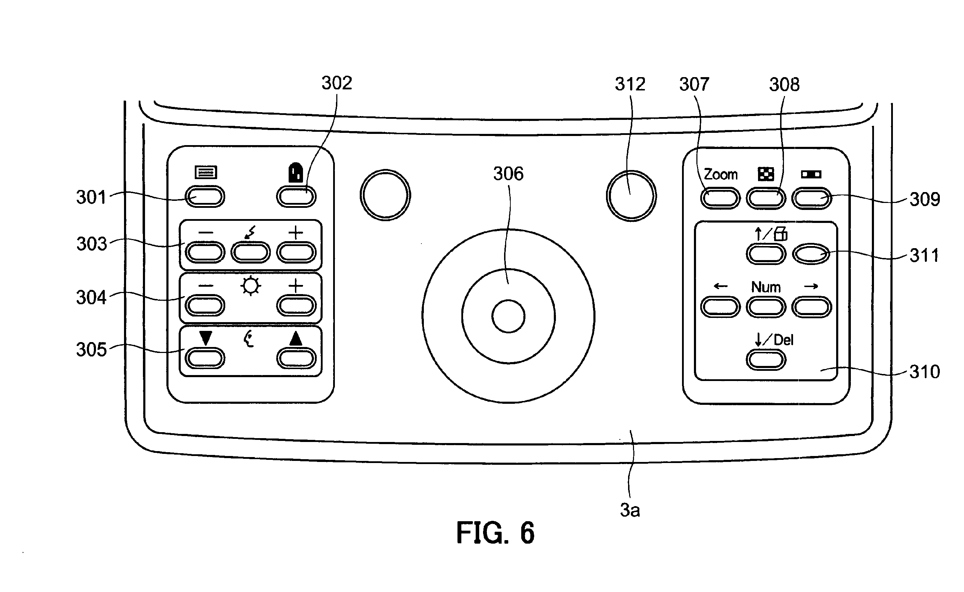

First, referring to Figs. 1 through 6, the configuration of the optical image measurement device according to a first embodiment of the present invention will be described. Fig. 1 shows one example of the entire configuration of a fundus oculi observation device 1 having a function as the optical image measurement device according to this embodiment. Fig. 2 shows one example of the configuration of a scan unit 141 in a retinal camera unit 1A. Fig. 3 shows one example of the configuration of an OCT unit 150. Fig. 4 shows one example of the hardware configuration of an arithmetic control unit 200. Fig. 5 shows one example of the configuration of a control system of the fundus oculi observation device 1. Fig. 6 shows one example of the configuration of an operation panel 3a disposed to the retinal camera unit 1A.

### [Entire Configuration]

The fundus oculi observation device 1 related to this embodiment comprises a retinal camera unit 1A, an OCT unit 150, and an arithmetic control unit 200 as shown in Fig.1. The retinal camera unit 1A has almost the same optical system as the conventional retinal cameras for photographing 2-diensional images of the fundus oculi surface. The OCT unit 150 houses an optical system that functions as an optical image measurement device. The arithmetic control unit 200 is equipped with a computer for executing various types of arithmetic processes, control processes, or the like.

To the OCT unit 150, one end of a connection line 152 is attached. A connector part 151 for connecting the connection line 152 to the retinal camera unit 1A is attached to the other end of the connection line 152. A conductive optical fiber runs through inside the connection line 152. Thus, the OCT unit 150 and the retinal camera unit 1 A are optically connected via the connection line 152.

### [Configuration of Retinal Camera Unit]

The retinal camera unit 1A is used for forming a 2-dimensional image of the surface of a fundus oculi of an eye, based on optically obtained data (data detected by the imaging devices 10 and 12). Herein, the "2-dimensional image of the surface of the fundus oculi" refers to a color or monochrome image of the surface of the fundus oculi having been photographed, a fluorescent image (a fluorescein angiography image, an indocyanine green fluorescent image, etc.), and the like. As well as the conventional retinal camera, the retinal camera unit 1A is provided with an illumination optical system 100 that illuminates a fundus oculi Ef of an eye E, and an imaging optical system 120 that guides the fundus oculi reflection light of the illumination light to the imaging device 10.

Although the details will be described later, the imaging device 10 in the imaging optical system 120 of this embodiment detects the illumination light having a wavelength in the near-infrared region. Moreover, this imaging optical system 120 is further provided with the imaging device 12 for detecting the illumination light having a wavelength in the visible region. Moreover, this imaging optical system 120 guides a signal light coming from the OCT unit 150 to the fundus oculi Ef, and guides the signal light passed through the fundus oculi Ef to the OCT unit 150.

The illumination optical system 100 comprises: an observation light source 101; a condenser lens 102; an imaging light source 103; a condenser lens 104; exciter filters 105 and 106; a ring transparent plate 107; a mirror 108; an LCD (Liquid Crystal Display) 109; an illumination diaphragm 110; a relay lens 111; an aperture mirror 112; and an objective lens 113.

The observation light source 101 emits an illumination light having a wavelength of the visible region included in a range of, for example, about 400 nm thorough 700 nm. Moreover, the imaging light source 103 emits an illumination light having a wavelength of the near-infrared region included in a range of, for example, about 700 nm through 800 nm. The near-infrared light emitted from this imaging light source 103 is set so as to have a shorter wavelength than the light used by the OCT unit 150 (described later).

Further, the imaging optical system 120 comprises: an objective lens 113; an aperture mirror 112 (an aperture 112a thereof); an imaging diaphragm 121; barrier filters 122 and 123; a variable magnifying lens 124; a relay lens 125; an imaging lens 126; a dichroic mirror 134; a field lens 128; a half mirror 135; a relay lens 131; a dichroic mirror 136; an imaging lens 133; the imaging device 10 (image pick-up element 10a); a reflection mirror 137; an imaging lens 138; the imaging device 12 (image pick-up element 12a); a lens 139; and an LCD 140.

The dichroic mirror 134 is configured to reflect the fundus oculi reflection light (having a wavelength included in a range of about 400 nm through 800 nm) of the illumination light from the illumination optical system 100, and transmit a signal light LS (having a wavelength included in a range of, for example, about 800 nm through 900 nm; described later) from the OCT unit 150.

Further, the dichroic mirror 136 is configured to transmit the illumination light having a wavelength of the visible region from the illumination optical system 100 (a visible light having a wavelength of about 400 nm through 700 nm emitted from the observation light source 101), and reflect the illumination light having a wavelength of the near-infrared region (a near-infrared light having a wavelength of about 700 nm through 800 nm emitted from the imaging light source 103).

On the LCD 140, a fixation target (internal fixation target) or the like for fixing the eye E is displayed. The light from this LCD 140 is reflected by the half mirror 135 after being converged by the lens 139, and is reflected by the dichroic mirror 136 through the field lens 128. Then, the light passes through the imaging lens 126, the relay lens 125, the variable magnifying lens 124, the aperture mirror 112 (aperture 112a thereof), the objective lens 113 and the like, and enters the eye E. Consequently, an internal fixation target or the like is projected in the fundus oculi Ef of the eye E.

The image pick-up element 10a is an image pick-up element such as a CCD (Charge Coupled Device) and a CMOS (Complementary metal Oxide Semiconductor) installed in the imaging device 10 such as a TV camera, and is particularly used for detecting light having a wavelength of the near-infrared region. In other words, the imaging device 10 is an infrared TV camera for detecting near-infrared light. The imaging device 10 outputs video signals as a result of detection of the near-infrared light.

A touch panel monitor 11 displays a 2-dimensional image (a fundus oculi image Ef') of the surface of the fundus oculi Ef, based on the video signals. The video signals are sent to the arithmetic control unit 200, and the fundus oculi image is displayed on the display (described later).

At the time of imaging of the fundus oculi by the imaging device 10, for example, the illumination light emitted from the imaging light source 103 of the illumination optical system 100 and having a wavelength of the near-infrared region is used.

On the other hand, the image pick-up element 12a is an image pick-up element such as a CCD and a CMOS installed in the imaging device 12 such as a TV camera, and is particularly used for detecting light having a wavelength of the visible region (that is, the imaging device 12 is a TV camera for detecting visible light). The imaging device 12 outputs video signals as a result of detection of the visible light.

The touch panel monitor 11 displays a 2-dimensional image (fundus oculi image Ef') of the surface of the fundus oculi Ef, based on the video signals. The video signals are sent to the arithmetic control unit 200, and the fundus oculi image Ef' is displayed on the display (described later).

At the time of imaging of the fundus oculi by the imaging device 12, for example, the illumination light emitted from the observation light source 101 of the illumination optical system 100 and having a wavelength of the visible region is used.

The retinal camera unit 1A is provided with a scan unit 141 and a lens 142. The scan unit 141 includes a component for scanning at an application position of the fundus oculi Ef with light emitted from the OCT unit (signal light LS; described later). The scan unit 141 functions as one example of the "scanner" of the present invention.

The lens 142 makes the signal light LS guided from the OCT unit 150 through the connection line 152 enter the scan unit 141 in the form of a parallel light flux. Moreover, the lens 142 acts so as to converge the fundus oculi reflection light of the signal light LS passed through the scan unit 141.

Fig. 2 shows one example of a specific configuration of the scan unit 141. The scan unit 141 comprises Galvano mirrors 141A and 141 B, and reflection mirrors 141C and 141D.

The Galvano mirrors 141A and 141B are reflection mirrors disposed so as to be rotatable about rotary shafts 141a and 141b, respectively. The Galvano mirrors 141A and 141B are rotated about the rotary shafts 141a and 141b, respectively, by a drive mechanism described later (mirror drive mechanisms 241 and 242 shown in Fig. 5), whereby the orientations of reflection surfaces thereof (faces reflecting the signal light LS), namely, the positions of the Galvano mirrors 141A and 141 B are changed, respectively.

The rotary shafts 141a and 141b are arranged so as to be orthogonal to each other. In Fig. 2, the rotary shaft 141a of the Galvano mirror 141A is arranged in parallel to the paper face of Fig. 2, whereas the rotary shaft 141b of the Galvano mirror 141B is arranged so as to be orthogonal to the paper face of Fig. 2.

That is to say, the Galvano mirror 141B is formed so as to be rotatable in the directions indicated by an arrow pointing in both directions in Fig. 2, whereas the Galvano mirror 141 A is formed so as to be rotatable in the directions orthogonal to the arrow pointing in both the directions. Consequently, the pair of Galvano mirrors 141A and 141B act so as to change the reflecting directions of the signal light LS to directions orthogonal to each other. As seen from Figs. 1 and 2, scan with the signal light LS is performed in the x direction when the Galvano mirror 141A is rotated, and scan with the signal light LS is performed in the y direction when the Galvano mirror 141B is rotated.

The signal lights LS reflected by the Galvano mirrors 141A and 141B are reflected by reflection mirrors 141C and 141D, thereby traveling in the same directions as having entered into the Galvano mirror 141A.

As described before, the conductive optical fiber 152a runs through the inside of the connection line 152, and an end face 152b of the optical fiber 152a is arranged facing the lens 142. The signal light LS emitted from this end face 152b travels while expanding its beam diameter toward the lens 142. The light is converged into a parallel light flux by this lens 142. On the contrary, the signal light LS passed through the fundus oculi Ef is converged toward the end face 152b by the lens 142, and guided to the optical fiber 152a.

### [Configuration of OCT Unit]

Next, the configuration of the OCT unit 150 will be described referring to Fig. 3. The OCT unit 150 is a device configured to form a tomographic image of the fundus oculi based on optically obtained data (data detected by a CCD 184 described later).

The OCT unit 150 has almost the same optical system as the conventional optical image measurement device. That is, the OCT unit 150 has: an interferometer that splits the light emitted from the light source into a reference light and a signal light and generates interference-light by superposing the reference light passed through a reference object and the signal light passed through a measurement object (fundus oculi Ef); and a part configured to detect this interference-light and output signals as the result of the detection (detection signals) toward the arithmetic control unit 200. The arithmetic control unit 200 forms a tomographic image of the measurement object (fundus oculi Ef), by analyzing the detection signals.

A low-coherence light source 160 is composed of a broadband light source, such as a super luminescent diode (SLD) and a light emitting diode (LED), configured to emit a low-coherence light L0. This low-coherence light L0 is, for example, a light that has a wavelength of the near-infrared region and has a time-wise coherence length of approximately several tens of micrometers. The low-coherence light source 160 corresponds to one example of the "light source" of the present invention.

The low-coherence light L0 has a longer wavelength than the illumination light (wavelength: about 400 nm through 800 nm) of the retinal camera unit 1A, for example, a wavelength included in a range of about 800 nm through 900 nm.

The low-coherence light L0 emitted from the low-coherence light source 160 is guided to an optical coupler 162 through an optical fiber 161. The optical fiber 161 is composed of, for example, a single mode fiber or a PM (Polarization maintaining) fiber. The optical coupler 162 splits this low-coherence light L0 into a reference light LR and the signal light LS.

Although the optical coupler 162 acts as both a part (splitter) for splitting light and a part (coupler) for superposing lights, it will be herein referred to as an "optical coupler" idiomatically.

The reference light LR generated by the optical coupler 162 is guided by an optical fiber 163 composed of a single mode fiber or the like, and emitted from the end face of the fiber. The emitted reference light LR is converged into a parallel light flux by a collimator lens 171, passed through a glass block 172 and a density filter 173, and then reflected by a reference mirror 174 (reference object).

The reference light LR reflected by the reference mirror 174 is converged to the fiber end face of the optical fiber 163 by the collimator lens 171 again trough the density filter 173 and the glass block 172. The converged reference light LR is guided to the optical coupler 162 through the optical fiber 163.

The glass block 172 and the density filter 173 act as a delaying part for making the optical path lengths (optical distances) of the reference light LR and the signal light LS coincide, and also as a dispersion correction part for making the dispersion characteristics of the reference light LR and the signal light LS coincide.

Further, the density filter 173 also acts as a dark filter for reducing the amount of the reference light, and is composed of a rotating ND (neutral density) filter, for example. This density filter 173 acts so as to change the reduction amount of the reference light LR by being rotary driven by a drive mechanism including a drive unit such as a motor (a density filter drive mechanism 244 described later; refer to Fig. 5). Consequently, it is possible to change the amount of the reference light LR contributing to generation of the interference-light LC.

Further, the reference mirror 174 is configured so as to move in the traveling direction (the direction of the arrow pointing both sides shown in Fig. 3) of the reference light LR. With this configuration, it is possible to ensure the optical path length of the reference light LR according to the axial length of the eye E, etc. Moreover, it is possible to capture an image of any depth position of the fundus oculi Ef, by moving the reference mirror 174. The reference mirror 174 is moved by a drive mechanism (a reference mirror driving mechanism 243 described later; refer to Fig. 5) including a driver such as a motor.

On the other hand, the signal light LS generated by the optical coupler 162 is guided to the end of the connection line 152 through an optical fiber 164 composed of a single mode fiber or the like. The conductive optical fiber 152a runs inside the connection line 152. Herein, the optical fiber 164 and the optical fiber 152a may be composed of a single optical fiber, or may be jointly formed by connecting the end faces of the respective fibers. In either case, it is sufficient as far as the optical fiber 164 and 152a are configured to be capable of transferring the signal light LS between the retinal camera unit 1A and the OCT unit 150.

The signal light LS is guided through the inside of the connection line 152 and led to the retinal camera unit 1A. Then, the signal light LS enters into the eye E through the lens 142, the scan unit 141, the dichroic mirror 134, the imaging lens 126, the relay lens 125, the variable magnifying lens 124, the imaging diaphragm 121, the aperture 112a of the aperture mirror 112, and the objective lens 113. The barrier filter 122 and 123 are retracted from the optical path in advance, respectively, when the signal light LS is made to enter the eye E.

The signal light LS having entered the eye E forms an image on the fundus oculi (retina) Ef and is then reflected. At this moment, the signal light LS is not only reflected on the surface of the fundus oculi Ef, but also scattered at the refractive index boundary after reaching the deep area of the fundus oculi Ef. As a result, the signal light LS passed through the fundus oculi Ef is a light containing information reflecting the state of the surface of the fundus oculi Ef and information reflecting the state of backscatter at the refractive index boundary of the deep area tissue of the fundus oculi Ef. This light may be simply referred to as "fundus oculi reflection light of the signal light LS."

The fundus oculi reflection light of the signal light LS travels reversely on the above path within the retinal camera unit 1A, and is converged at the end face 152b of the optical fiber 152a. Then, the signal light LS enters into the OCT unit 150 through the optical fiber 152a, and returns to the optical coupler 162 through the optical fiber 164.

The optical coupler 162 superimposes the signal light LS returning through the fundus oculi Ef and the reference light LR reflected by the reference mirror 174, thereby generating the interference-light LC. The generated interference-light LC is guided into a spectrometer 180 through an optical fiber 165 composed of a single mode fiber or the like.

Herein, although a Michelson-type interferometer is adopted in this embodiment, for instance, a Mach Zender type, etc. and any type of interferometer may be adopted appropriately. The "interference-light generator" related to the present invention comprises, for example, an optical coupler 162, an optical member on the optical path of the signal light LS (i.e., an optical member placed between the optical coupler 162 and the fundus oculi Ef), and an optical member on the optical path of the reference light LR (i.e., an optical member placed between the optical coupler 162 and the reference mirror 174), and specifically, comprises an interferometer equipped with the optical coupler 162, the optical fibers 163, 164, and the reference mirror 174.

The spectrometer 180 comprises a collimator lens 181, a diffraction grating 182, an image-forming lens 183, and a CCD 184. The diffraction grating 182 in this embodiment is a transmission-type diffraction grating that transmits light; however, needless to say, a reflection-type diffraction grating that reflects light may also be used. Moreover, needless to say, it is also possible to adopt, in place of the CCD 184, other photo-detecting elements.

The interference light LC having entered the spectrometer 180 is split (resolved into spectra) by the diffraction grating 182 after converged into a parallel light flux by the collimator lens 181. The split interference light LC forms an image on the image pick-up surface of the CCD 184 by the image-forming lens 183. The CCD 184 detects the respective spectra of the interference light LC and converts to electrical detection signals, and outputs the detection signals to the arithmetic control unit 200. The CCD 184 functions as the "detector" of the present invention.

### [Configuration of Arithmetic Control Unit]

Next, the configuration of the arithmetic control unit 200 will be described. The arithmetic control unit 200 performs a process of analyzing detection signals inputted from the CCD 184 of the spectrometer 180 of the OCT unit 150 and forming a tomographic image of the fundus oculi Ef of the eye E. The analysis method is the same as the conventional technique of Fourier Domain OCT.

Further, the arithmetic control unit 200 performs a process of forming (image data of) a 2-dimensional image showing the state of the surface (retina) of the fundus oculi Ef , based on the video signals outputted from the imaging devices 10 and 12 of the retinal camera unit 1A.

Furthermore, the arithmetic control unit 200 executes control of each part of the retinal camera unit 1A and the OCT unit 150.

The arithmetic control unit 200 executes as control of the retinal camera unit 1A, for example: control of emission of illumination light by the observation light source 101 or the imaging light source 103; control of insertion/retraction operations of the exciter filters 105 and 106 or the barrier filters 122 and 123 to/from the optical path; control of the operation of a display device such as the LCD 140; control of shift of the illumination diaphragm 110 (control of the diaphragm value); control of the diaphragm value of the imaging diaphragm 121 ; and control of shift of the variable magnifying lens 124 (control of the magnification). Moreover, the arithmetic control unit 200 executes control of the operation of the Galvano mirrors 141A and 141 B inside the scan unit 141 (operation of changing the directions of the reflection faces).

Further, the arithmetic control unit 200 executes as control of the OCT unit 150, for example: control of emission of the low-coherence light L0 by the low-coherence light source 160; control of shift of the reference mirror 174; control of the rotary operation of the density filter 173 (operation of changing the reduction amount of the reference light LR); and control of the accumulated time of the CCD 184.

One example of the hardware configuration of the arithmetic and control unit 200 that acts as described above will be described referring to Fig. 4.

The arithmetic and control unit 200 is provided with the same hardware configuration as that of a conventional computer. To be specific, the arithmetic and control unit 200 comprises: a microprocessor 201 (CPU, MPU, etc.), a RAM202, a ROM203, a hard disk drive (HDD) 204, a keyboard 205, a mouse 206, a display 207, an image forming board 208, and a communication interface (I/F) 209. These parts are connected via a bus 200a.

The microprocessor 201 comprises a CPU (Central Processing Unit), an MPU (Micro Processing Unit) or the like, and executes operations characteristic to this embodiment, by loading a control program 204a stored in the hard disk drive 204, onto the RAM 202.

Further, the microprocessor 201 executes control of each part of the device described above, various arithmetic processes, etc. Moreover, the microprocessor 201 executes control of each part of the device corresponding to an operation signal from the keyboard 205 or the mouse 206, control of a display process by the display 207, and control of a transmission/reception process of various data, control signals and so on by the communication interface 209.

The keyboard 205, the mouse 206 and the display 207 are used as user interfaces in the fundus oculi observation device 1. The keyboard 205 is used as, for example, a device for typing letters, figures, etc. The mouse 206 is used as a device for performing various input operations to the display screen of the display 207.

Further, the display 207 is any display device composed of an LCD, a CRT (Cathode Ray Tube) display or the like. The display 207 displays various images of the fundus oculi Ef formed by the fundus oculi observation device 1, and displays various screens such as an operation screen and a set-up screen.

The user interface of the fundus oculi observation device 1 is not limited to the above configuration, and may be configured by using any user interface having a function of displaying and outputting various information, and a function of inputting various information and operating the device, such as a track ball, a control lever, a touch panel type of LCD, and a control panel for ophthalmology examinations.

The image forming board 208 is a dedicated electronic circuit for a process of forming (image data of) images of the fundus oculi Ef of the eye E. This image forming board 208 is provided with a fundus oculi image forming board 208a and an OCT-image forming board 208b.

The fundus oculi image forming board 208a is a dedicated electronic circuit that operates to form image data of fundus oculi images based on the video signals from the imaging device 10 and the imaging device 12 of the retinal camera unit 1A.

Further, the OCT-image forming board 208b is a dedicated electronic circuit that operates to form image data of tomographic images of the fundus oculi Ef, based on the detection signals from the CCD 184 of the spectrometer 180 in the OCT unit 150.

By providing the image forming board 208, it is possible to increase the processing speed for forming image data of fundus oculi images and tomographic images.

The communication interface 209 performs a process of sending control signals from the microprocessor 201, to the retinal camera unit 1A or the OCT unit 150. Moreover, the communication interface 209 performs a process of receiving video signals from the imaging devices 10 and 12 of the retinal camera unit 1 A and detection signals from the CCD 184 of the OCT unit 150, and inputting the signals to the image forming board 208. At this time, the communication interface 209 operates to input the video signals from the imaging devices 10 and 12, to the fundus oculi image forming board 208a, and input the detection signal from the CCD 184, to the OCT-image forming board 208b.

Further, in a case where the arithmetic and control unit 200 is connected to a network such as a LAN (Local Area Network) and the Internet, it is possible to configure so as to be capable of data communication via the network, by providing the communication interface 209 with a network adapter like a LAN card or communication equipment like a modem. In this case, by mounting a server accommodating the control program 204a on the network, and at the same time, configuring the arithmetic and control unit 200 as a client terminal of the server, it is possible to cause the fundus oculi observation device 1 to execute the operation according to the present invention.

### [Configuration of Control System]

Next, the configuration of the control system of the fundus oculi observation device 1 will be described referring to Fig. 5 and Fig. 6. Fig. 5 is a block diagram showing a part related to the operations and processes according to the present invention particularly selected from among constituents composing the fundus oculi observation device 1. Fig. 6 shows one example of the configuration of the operation panel 3a disposed to the retinal camera unit 1A.

### (Controller)

The control system of the fundus oculi observation device 1 is configured mainly having a controller 210 of the arithmetic and control unit 200 shown in Fig. 5. The controller 210 comprises the microprocessor 201, the RAM202, the ROM203, the hard disk drive 204 (control program 204a), and the communication interface 209.

The controller 210 executes the aforementioned various controlling processes through the microprocessor 201 operating based on the control program 204a. In specific, for the retinal camera unit 1A, the controller 210 executes control of the mirror drive mechanisms 241 and 242 for changing the positions of the Galvano mirrors 141A and 141B, and functions as one example of the "controller" of the present invention.

Further, for the OCT unit 150, the controller 210 performs control of the low-coherence light source 160 and the CCD 184, control of the density filter drive mechanism 244 for rotating the density filter 173, control of the reference-mirror driving mechanism 243 for moving the reference mirror 174 in the traveling direction of the reference light LR, etc.

Furthermore, the controller 210 performs control for causing the display 240A of the user interface (UI) 240 to display two kinds of images photographed by the fundus oculi observation device 1: that is, a 2-dimensional image of the surface of the fundus oculi Ef obtained by the retinal camera unit 1A, and a tomographic image of the fundus oculi Ef formed based on the detection signals obtained by the OCT unit 150. These images may be displayed on the display 240A separately, or may be displayed side by side simultaneously.

### (Scanning line setting part)

The controller 210 is provided with a scanning line setting part 211. The scanning line setting part 211 sets a scanning line that becomes a scan position of the signal light LS (described below).

To be specific, the scanning line setting part 211 operates when a region on the fundus oculi Ef is designated, and sets, in this designated region, scanning lines of a number depending on the size of the designated region. The set number of the scanning lines is a number that allows formation of a 3-dimensional image of the fundus oculi Ef in the designated region.

The relation between the size of the designated region and the number of the scanning lines will be described. At the time of formation of a 3-dimensional image, an interpolation process is applied to a plurality of tomographic images, so that the adjacent tomographic images need to be close to each other. In other words, if the adjacent tomographic images are distant from each other by a predetermined distance or more, it is impossible to favorably apply the interpolation process, and thus, it is impossible to form a 3-dimensional image. Here, the tomographic images are formed along the scanning lines. Therefore, in order to acquire a 3-dimensional image, the adjacent scanning lines needs to be close to each other.

The scanning line setting part 211 determines the number of the scanning lines so that the scanning lines are arranged so as to enable favorable application of the interpolation process for forming a 3-dimensional image, and also sets the positions of these scanning lines. Information on the interval between the scanning lines that enables favorable application of the interpolation process is prestored in a hard disk drive 204 or the like.

### (Image Forming Part)

An image forming part 220 performs a process of forming image data of the fundus oculi image based on the video signals from the imaging devices 10 and 12 of the retinal camera unit 1A, and a process of forming image data of the tomographic images of the fundus oculi Ef based on the detection signals from the CCD 184 of the OCT unit 150.

The imaging forming part 220 comprises the imaging forming board 208 and the communication interface 209. In this specification, "image" may be identified with "image data" corresponding thereto.

### (Image Processor)

The image processor 230 applies various image processing and analysis process to image data of images formed by the image forming part 220. For example, the image processor 230 executes various correction processes such as brightness correction and dispersion correction of images.

### (3-dimensional image forming part)

The image processor 230 is provided with a 3-dimensional image forming part 231. The 3-dimensional image forming part 231 applies, to tomographic images formed by the image-forming part 220, an interpolation process of interpolating pixels between the tomographic images, thereby forming image data of a 3-dimensional image of the fundus oculi Ef.

Herein, image data of a 3-dimensional image is image data made by assigning pixel values to each of a plurality of voxels arranged 3-dimensionally, and is referred to as volume data, voxel data, and the like. When displaying an image based on volume data, the image processor 230 operates to apply a rendering process (such as volume rendering and MIP (Maximum Intensity Projection)) to this volume data and form image data of a pseudo 3-dimensional image seen from a specified viewing direction. On a display device such as the display 207, the pseudo 3-dimensional image based on the image data is displayed.

Moreover, the 3-dimensional image forming part 231 is also capable of forming stack data of a plurality of tomographic images. The stack data is image data that is obtained by arraying a plurality of tomographic images that have been obtained along a plurality of scanning lines, based on the positional relationship between the scanning lines.

Herein, the image forming part 220 (OCT-image forming board 208b) and the 3-dimensional image forming part 231 function as one example of the "image forming part" of the present invention.

The image processor 230 that operates as described above comprises the microprocessor 201, the RAM 202, the ROM 203, and the hard disk drive 204 (control program 204a).

### (User Interface)

The user interface (UI) 240 comprises the display 240A and an operation part 240B. The display 240A is composed of a display device such as the display 207. Further, the operation part 240B is composed of an input device or an operation device such as the keyboard 205 and the mouse 206. The operation part 240B functions as an example of the "designating part," and also functions as an example of the "operation part" in the present invention.

### (Operation Panel)

The operation panel 3a of the retinal camera unit 1 A will be described. The operation panel 3a is arranged on the platform (not shown) of the retinal camera unit 1A, for example.

The operation panel 3a is provided with an operation part used to instruct an operation for capturing a 2-dimensional image of the surface of the fundus oculi Ef, and an operation part used to instruct an operation for capturing a tomographic image of the fundus oculi Ef.

Placement of the operation panel 3a makes it possible to execute an operation for capturing a fundus oculi image Ef' and an operation for capturing a tomographic image in the same manner as when operating a conventional retinal camera.

As shown in Fig. 6, the operation panel 3a is provided with, for example, a menu switch 301, a split switch 302, an imaging light amount switch 303, an observation light amount switch 304, a jaw holder switch 305, a photographing switch 306, a zoom switch 307, an image switching switch 308, a fixation target switching switch 309, a fixation target position adjusting switch 310, a fixation target size switching switch 311, and a mode switching knob 312.

The menu switch 301 is a switch operated to display a certain menu screen for a user to select and designate various menus (such as an imaging menu for imaging a 2-dimensional image of the surface of the fundus oculi Ef, a tomographic image and 3-dimentional image the like, and a setting menu for entering various settings).

When this menu switch 301 is operated, the operation signal is inputted to the controller 210. The controller 210 causes the touch panel monitor 11 or the display 240A to display a menu screen, in response to the input of the operation signal. A controller (not shown) may be provided in the retinal camera unit 1A, whereby the controller causes the touch panel monitor 11 to display the menu screen.

The split switch 302 is a switch operated to switch the light on and off of the split bright line for focusing (e.g., see Japanese Unexamined Patent Application Publication JP-A 9-66031. Also referred to as split target, split mark and so on.). The configuration for projecting this split bright line onto the eye E (split bright line projection part) is housed, for example, in the retinal camera unit 1A (not shown in Fig. 1).

When this split switch 302 is operated, the operation signal is inputted to the controller 210 (or the aforementioned controller inside the retinal camera unit 1A; the same hereinafter). The controller 210 projects the split bright line onto the eye E by controlling the split bright line projection part, in response to the input of this operation signal.

The imaging light amount switch 303 is a switch operated to adjust the emitted light amount of the imaging light source 103 (photographing light amount) depending on the state of the eye E (such as the degree of opacity of the lens). This imaging light amount switch 303 is provided with, for example, a photographing light amount increasing switch "+" for increasing the photographing light amount, a photographing light amount decreasing switch "_'' for decreasing the photographing light amount, and a reset switch (a button in the middle) for setting the photographing light amount to a predetermined initial value (default value).

When one of the imaging light amount switches 303 is operated, the operation signal is inputted to the controller 210. The controller 210 controls the imaging light source 103 in response to the inputted operation signal and adjusts the photographing light amount.

The observation light amount switch 304 is a switch operated to adjust the emitted light amount (observation light amount) of the observation light source 101. The observation light amount switch 304 is provided with, for example, an observation light amount increasing switch "+'' for increasing the observation light amount, and an observation light amount decreasing switch "-'' for decreasing the observation light amount.

When one of the observation light amount switches 304 is operated, the operation signal is inputted to the controller 210. The controller 210 controls the observation light source 101 in response to the inputted operation signal and adjusts the observation light amount.

The jaw holder switch 305 is a switch to move the position of the jaw holder (not shown) of the retinal camera unit 1A. This jaw holder switch 305 is provided with, for example, an upward movement switch (upward triangle) for moving the jaw holder upward, and a downward movement switch (downward triangle) for moving the jaw holder downward.

When one of the jaw holder switches 305 is operated, the operation signal is inputted to the controller 210. The controller 210 controls a jaw holder movement mechanism (not shown) in response to the inputted operation signal and moves the jaw holder upward or downward.

The photographing switch 306 is a switch used as a trigger switch for capturing a 2-dimensional image of the surface of the fundus oculi Ef or a tomographic image of the fundus oculi Ef.

When the photographing switch 306 is operated in a state where a menu to photograph a 2-dimensional image is selected, the controller 210 that has received the operation signal controls the imaging light source 103 to emit photographing illumination light, and also causes the display 240A or the touch panel monitor 11 to display a 2-dimensional image of the surface of the fundus oculi Ef, based on the video signal outputted from the imaging device 10 having detected the fundus oculi reflection light.

On the other hand, when the imaging switch 306 is operated in a state where a menu to capture a tomographic image is selected, the controller 210 that has received the operation signal controls the low-coherence light source 160 to emit the low-coherence light L0, and also controls the Galvano mirrors 141 A and 141 B to scan the signal light LS. Moreover, the controller 210 causes the display 240A or the touch panel monitor 11 to display a tomographic image of the fundus oculi Ef formed by the image forming part 220 (and image processor 230), based on the detection signal outputted from the CCD 184 that has detected the interference light LC. When the menu for obtaining 3-dimensional image is selected, a 3-dimensional image of the fundus oculi Ef based on a plurality of similarly obtained tomographic images is displayed on the display 240A.

The zoom switch 307 is a switch operated to change the angle of view (zoom magnification) at the time of photographing of the fundus oculi Ef. Every time this zoom switch 307 is operated, the photographing angle is set alternately to 45 degrees and 22.5 degrees, for example.

When this zoom switch 307 is operated, the controller 210 that has received the operation signal controls a variable magnifying lens driving mechanism (not shown) to move the variable magnifying lens 124 in the optical axis direction of the imaging optical system 120, thereby changing the photographing angle of view.

The image switching switch 308 is a switch operated to switch displayed images. When the image switching switch 308 is operated in a state where a fundus oculi observation image (a 2-dimensioal image of the surface of the fundus oculi Ef based on the video signal from the imaging device 12) is displayed on the display 240A or the touch panel monitor 11, the controller 210 having received the operation signal controls the display 240A or touch panel monitor 11 to display the tomographic image of the fundus oculi Ef.

On the other hand, when the image-switching switch 308 is operated in a state where a tomographic image of the fundus oculi is displayed on the display 240A or the touch pane monitor 11, the controller 210 having received the operation signal controls the display 240A or the touch panel monitor 11 to display the fundus oculi observation image.

The fixation target-switching switch 309 is a switch operated to switch the position of the internal fixation target displayed by the LCD 140 (i.e. the projection position of the internal fixation target on the fundus oculi Ef). By operating this fixation target switching switch 309, the display position of the internal fixation target can be switched, for example, among "fixation position to capture the image of the peripheral region of the center of the fundus oculi (fixation position for fundus oculi center imaging)," "fixation position to capture the image of the peripheral region of macula lutea (fixation position for macula lutea imaging)'' and "fixation position to capture the image of the peripheral region of papilla (fixation position for papilla imaging)," in a circulative fashion.

In response to the operation signals from the fixation target-switching switch 309, the controller 210 causes the LCD 140 to display the internal fixation target in different positions on the display surface thereof. The display positions of the internal fixation target corresponding to the above three fixation positions, for example, can be preset based on clinical data, or can be set for each eye or for each image photographing in advance.

The fixation target position-adjusting switch 310 is a switch operated to adjust the display position of the internal fixation target. This fixation target position adjusting switch 310 is provided with, for example, an upward movement switch for moving the display position of the internal fixation target upward, a downward movement switch for moving it downward, a leftward movement switch for moving it leftward, a rightward movement switch for moving it rightward, and a reset switch for moving it to a predetermined initial position (default position).

Upon reception of the operation signal from either of these switches of the fixation target position-adjusting switch 310, the controller 210 controls the LCD 140 to move the display position of the internal fixation target, in response to the operation signal.

The fixation target size switching switch 311 is a switch operated to change the size of the internal fixation target. When this fixation target size switching switch 311 is operated, the controller 210 that has received the operation signal controls the LCD 140 to change the display size of the internal fixation target. The display size of the internal fixation target can be switched, for example, between "normal size" and "enlarged size,'' alternately. As a result, the size of the projection image of the fixation target projected onto the fundus oculi Ef is changed. Upon reception of the operation signal from the fixation target position adjusting switch 311, the controller 210 controls the LCD 140 to change the display size of the internal fixation target, in response to the operation signal.

The mode-switching knob 312 is a knob rotationally operated to select various imaging modes. The imaging modes are, for example, a fundus oculi imaging mode to obtain a 2-dimensional image of the fundus oculi Ef, a B-scan mode to perform B-scan of the signal light LS, an radial scanning mode for radially scanning the signal light LS, and a 3-dimensional scan mode to scan with the signal light LS 3-dimensionally. In addition, the mode-switching knob 312 may be configured so as to be capable of selecting a replay mode to replay and display a captured 2-dimensional image or tomographic image of the fundus oculi Ef. In addition, it may be configured so as to be capable of selecting a imaging mode to control so that imaging the fundus oculi Ef would be performed immediately after scanning of the signal light LS. Control of each part of the device for causing the fundus oculi observation device 1 to execute the operation corresponding to the each mode is executed by the controller 210.

### [Signal Light Scanning]

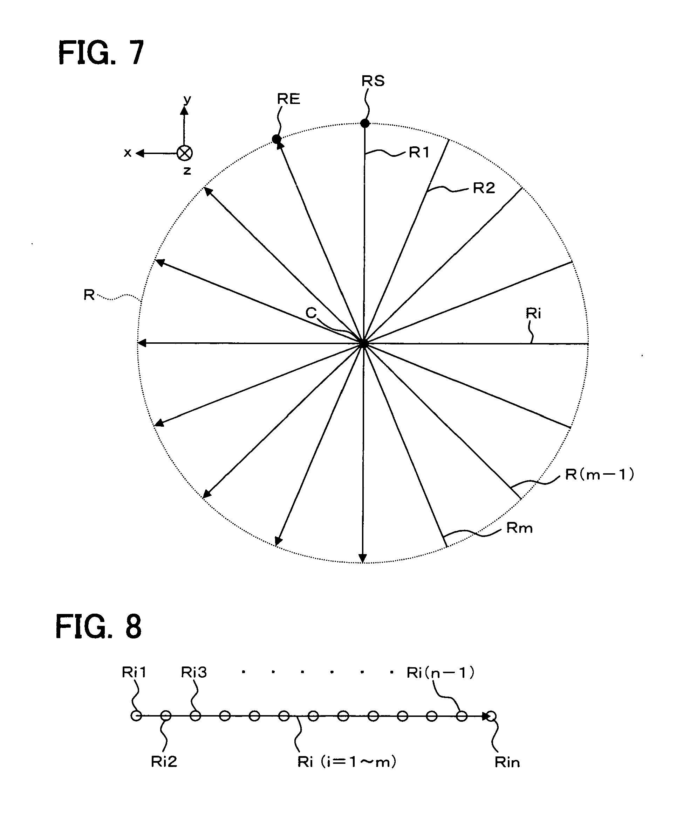

Hereinafter, scan with the signal light LS will be described referring to Fig. 7 and Fig. 8. Scanning lines as shown in Fig. 7 and Fig. 8 are not actually set on the fundus oculi Ef, but are virtual lines representing the trajectory of a target position of the signal light LS. In addition, each of the scanning lines also has significance as a line that indicates a cross-sectional position of a tomographic image, as described below.

Scanning of the signal light LS is performed by changing the positions (directions of the reflecting surfaces) of the Galvano mirrors 141A and 141B of the scan unit 141 in the retinal camera unit 1A. By controlling the mirror drive mechanisms 241 and 242 respectively to change the directions of the reflecting surfaces of the Galvano mirrors 141A and 141B respectively, the controller 210 scans the application position of the signal light LS on the fundus oculi Ef.

When the facing direction of the reflecting surface of the Galvano mirror 141 A is changed, the signal light LS is scanned in the horizontal direction (x-direction in Fig. 1) on the fundus oculi Ef. Whereas, when the facing direction of the reflecting surface of the Galvano mirror 141B is changed, the signal light LS is scanned in the vertical direction (y-direction in Fig. 1) on the fundus oculi Ef. Further, by changing the facing directions of the reflecting surfaces of both the Galvano mirrors 141A and 141B simultaneously, it is possible to scan the signal light LS in the composed direction of the x-direction and y-direction. That is, by controlling these two Galvano mirrors 141A and 141B, it is possible to scan the signal light LS in any direction on the x-y plane.

Fig. 7 shows one example of the feature of scanning of the signal light LS for forming images of the fundus oculi Ef. Fig. 7 shows one example of the feature of scanning of the signal light LS, when the fundus oculi Ef is seen from a direction that the signal light LS enters the eye E (that is, seen from -z side toward +z side in Fig. 1). Further, Fig. 8 shows one example of the feature of arrangement of scanning points (positions at which image measurement is carried out; target positions of the signal light LS) on each of the scanning lines.

As shown in Fig. 7, scan is performed with the signal light LS in a scanning region R having a shape of, for example, a circle. Here, the shape of the scanning region is not limited to a circle, but may be any shape such as a rectangle. The scanning region R is set on the fundus oculi Ef (on the fundus oculi image Ef'), for example, through a dragging operation with a mouse 206.

A plurality (m number) of scanning lines R1 through Rm, which are radially arranged, are set in the scanning region R. The respective scanning lines Ri are set so as to intersect each other at a predetermined position (center position) C. When scan is performed with the signal light LS along the respective scanning lines Ri, a detection signal of the interference light LC will be generated. Each of the scanning lines Ri (i=1 to m) is set by the scanning line setting part 211. An examiner may set the scanning lines Ri at any position by operating the operation part 240B. This setting operation can be performed through, for example, a dragging operation with the mouse 206.

On each scanning line Ri, as shown in Fig. 8, a plurality of (n number of) scanning points Ri1 through Rin are preset.

In order to execute the scanning shown in Figs. 7 and 8, the controller 210 firstly controls the Galvano mirrors 141A and 141B to set the target of the signal light LS entering into the fundus oculi Ef to a scan start position RS (scanning point R11) on the first scanning line R1. Subsequently, the controller 210 controls the low-coherence light source 160 to flush the low-coherence light L0, thereby making the signal light LS enter the scan start position RS. The CCD 184 receives the interference light LC based on the fundus oculi reflection light of this signal light LS at the scan start position RS, and outputs the detection signal to the controller 210.

Next, the controller 210 controls the Galvano mirrors 141A and 141B to set the incident target of the signal light LS to a scanning point R12, and makes the low-coherence light L0 flushed to make the signal light LS enter into the scanning point R12. The CCD 184 receives the interference light LC based on the fundus oculi reflection light of this signal light LS at the scanning point R12, and then outputs the detection signal to the controller 210.

Likewise, the controller 210 obtains detection signals outputted from the CCD 184 in response to the interference light LC for each scanning point, by flushing the low-coherence light L0 at each scanning point while shifting the incident target of the signal light LS from scanning point R13 to R14, ----, R1 (n-1), and R1n in order.

When measurement at the last scanning point R1n of the first scanning line R1 is finished, the controller 210 controls the Galvano mirrors 141A and 141 B to shift the incident target of the signal light LS to the first scanning point R21 of the second scanning line R2. Further, the controller 210 controls to perform this measurement on each scanning point R2j (j=1 through n) of this second scanning line R2.

Likewise, the measurement is conducted for each of the third scanning line R3, ----, the m-1th scanning line R(m-1), the mth scanning line Rm to obtain the detection signals corresponding to the respective scanning points. Symbol RE on a scanning line Rm is a scan end position corresponding to a scanning point Rmn.

Through this operation, m × n number of detection signals corresponding to m × n number of scanning points Rij (i=1 through m, j=1 through n) within the scanning region R are obtained. Hereinafter, a detection signal corresponding to the scanning point Rij may be represented by Dij.

Such interlocking control of the shift of scanning points and the emission of the low-coherence light L0 can be realized by synchronizing, for instance, timing for transmission of control signals to the mirror drive mechanisms 241 and 242 and timing for transmission of control signals (output request signals) to the low-coherence light source 160.

As described above, when each of the Galvano mirrors 141 A and 141 B is operated, the controller 210 stores the position of each scanning line Ri and the position of each scanning point Rij (coordinates on the x-y coordinate system) as information representing the content of the operation. This stored content (scanning point coordinate information) is used in an image forming process as in conventional one.

### [Image Processing]

Next, one example of a process on OCT images (tomography images of the fundus oculi Ef) by the image forming part 220 and the image processor 230 will be described.

As described above, the image forming part 220 executes the formation process of tomographic images of the fundus oculi Ef along each scanning line Ri (main scanning direction). Further, the image processor 230 executes the formation process of a 3-dimensional image of the fundus oculi Ef based on these tomographic images formed by the image forming part 220, etc.

The formation process of a tomographic image by the image forming part 220, as in the conventionally one, includes a 2-step arithmetic process. In the first step of the arithmetic process, based on a detection signal Dij corresponding to each scanning point Rij, an image in the depth-wise direction (z-direction in Fig. 1) of the fundus oculi Ef at the scanning point Rij is formed. The image in the depth-wise direction at the scanning point Rij is a 1-dimensional image passing through the scanning point Rij and extending in the z-direction.

In the second step of the arithmetic process, on each scanning line Ri, by lining up the images in the depth-wise direction at the n number of scanning points Ri1 through Rin, a tomographic image of the fundus oculi Ef along the scanning line Ri is formed. Then, the image forming part 220 determines the arrangement and the distance of the scanning points Ri1 through Rin referring to the positional information (scanning point coordinate information described before) of the scanning points Ri1 through Rin, and forms a tomographic image along this scanning line Ri.

Through the above process, m number of tomographic images corresponding to the scanning lines R1 through Rm can be obtained.

Next, a process of forming a 3-dimensional image of the fundus oculi Ef will be explained. A 3-dimensional image of the fundus oculi Ef is formed based on the m number of tomographic images obtained through the above arithmetic process. The 3-dimensional image forming part 231 performs a publicly known interpolation process of interpolating adjacent tomographic images to form a 3-dimensional image of the fundus oculi Ef. For this 3-dimensional image, a 3-dimensional coordinate system (x,y,z) is set, based on the positional information (the scanning point coordinate information) of each scanning point Rij and the z-coordinate in the depth-wise image.

Furthermore, based on this 3-dimensional image, the image processor 230 can form a tomographic image of the fundus oculi Ef at a cross-section in any direction other than the main scanning direction (x-direction). Once the cross-section is designated, the image processor230 determines the position of each scanning point (and/or an interpolated depth-wise image) on this designated cross-section, and extracts a depth-wise image at each determined position (and/or an interpolated depth-wise image), thereby forming a tomographic image of the fundus oculi Ef at the designated cross-section by arranging plural extracted depth-wise images.

### [Usage patterns]

A pattern of usage of the fundus oculi observation device 1 having the configuration as described above will be described. Below, three patterns of usage of the fundus oculi observation device 1 will be described. In Usage Pattern 1, an example of a process of forming a 3-dimensional image of a region depending on the interval between the scanning lines Ri will be described. In Usage Pattern 2, an example of a process of setting scanning lines depending on the size of a designated region and forming a 3-dimensional image will be described. In Usage Pattern 3, an example of a process of forming a 3-dimensional image of a region depending on a designated number of scanning lines will be described.

### (Usage Pattern 1)

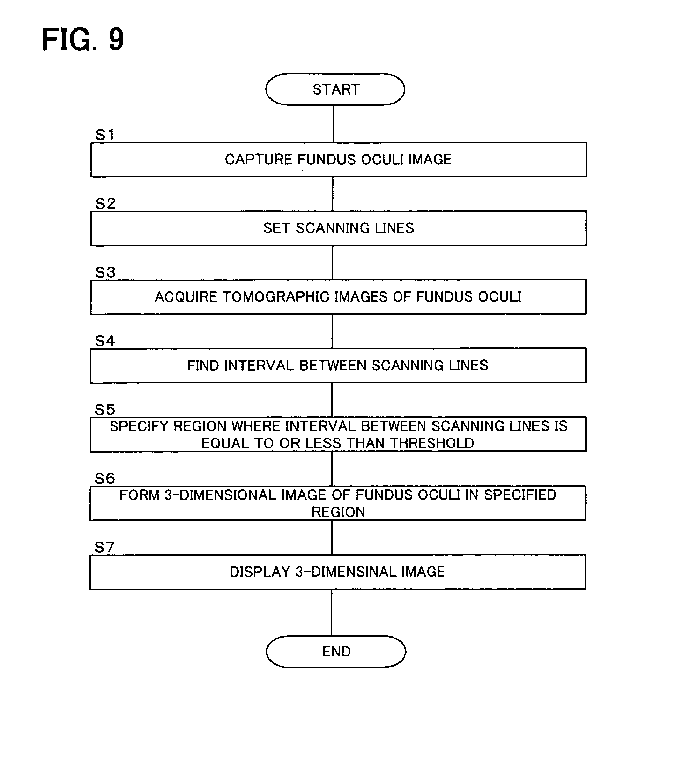

An example of a process of forming a 3-dimensional image of a region depending on the interval between the scanning lines Ri will be described referring to a flowchart shown in Fig. 9.

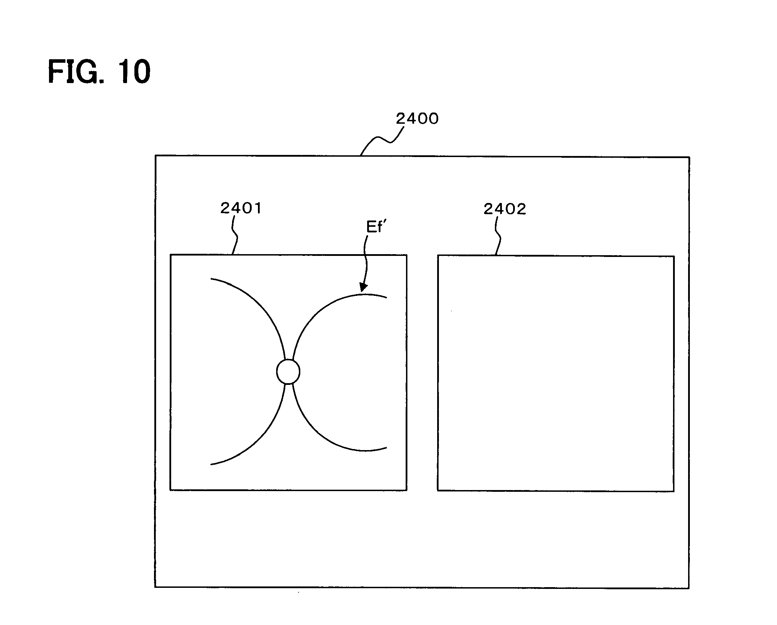

First, the fundus oculi observation device I captures a fundus oculi image Ef' of the eye E (S1). The controller 210 causes the display 240A to display the captured fundus oculi image Ef'. Fig. 10 shows an example of a display mode for the fundus oculi image Ef'.

A display screen 2400 of the display 240A has a fundus oculi image display region 2401 and an OCT image display region 2402. The fundus oculi image Ef' is displayed in the fundus oculi image display region 2401.

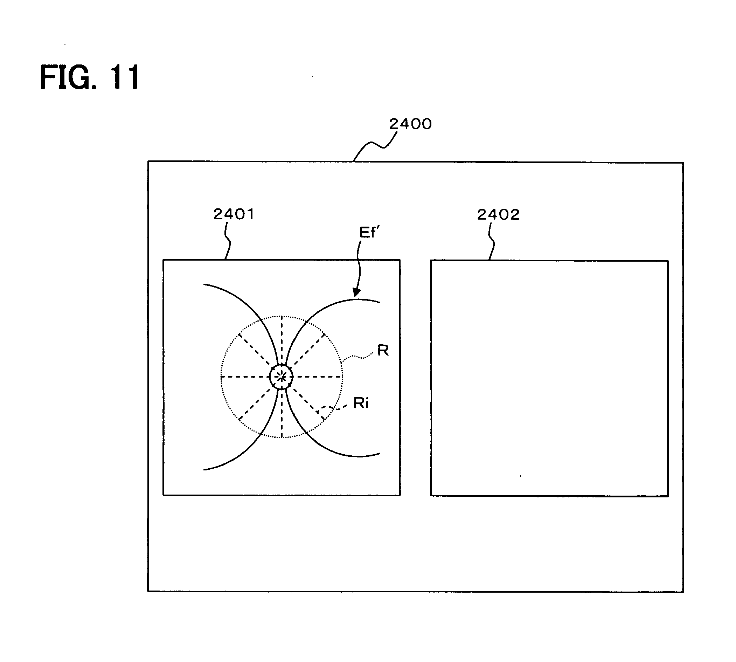

Next, the scanning lines Ri are set (S2). The scanning lines Ri are arranged radially so as to pass through the center position C (see Fig. 11). At this moment, a 2-dimensional region on the fundus oculi image Ef' in which the scanning lines Ri are set corresponds to a scanning region R.

Setting of the scanning lines Ri in Step 2 is performed by, for example, an examiner who operates the operation part 240B. The scanning lines Ri are set so as to pass through an attention site on the fundus oculi image Ef'. To be specific, the scanning lines Ri are set so that the center position C is placed in the center of optic papilla on the fundus oculi image Ef'. In this case, for example, the examiner firstly operates a mode-switching knob 312 to select a radial scan mode, and then clicks a mouse 26 or the like to designate the center position C on the fundus oculi image Ef' and also designate the scanning lines Ri at desired positions passing through this center position C.

When the setting of the scanning lines Ri is complete, a tomographic image of the fundus oculi Ef along each of the scanning lines Ri is acquired (S3). For this purpose, the examiner operates the photographing switch 306 to direct start of measurement. Upon receiving a signal from the photographing switch 306, the controller 210 controls the reference mirror driving mechanism 243 to place the reference mirror 174 at a position corresponding to a measurement depth, and then controls the low-coherence light source 160, the mirror-driving mechanisms 241 and 242 and so on to scan with the signal light LS along each of the scanning lines Ri. The image forming part 220 forms a tomographic image along each of the scanning lines Ri based on a detection signal from the CCD 184.

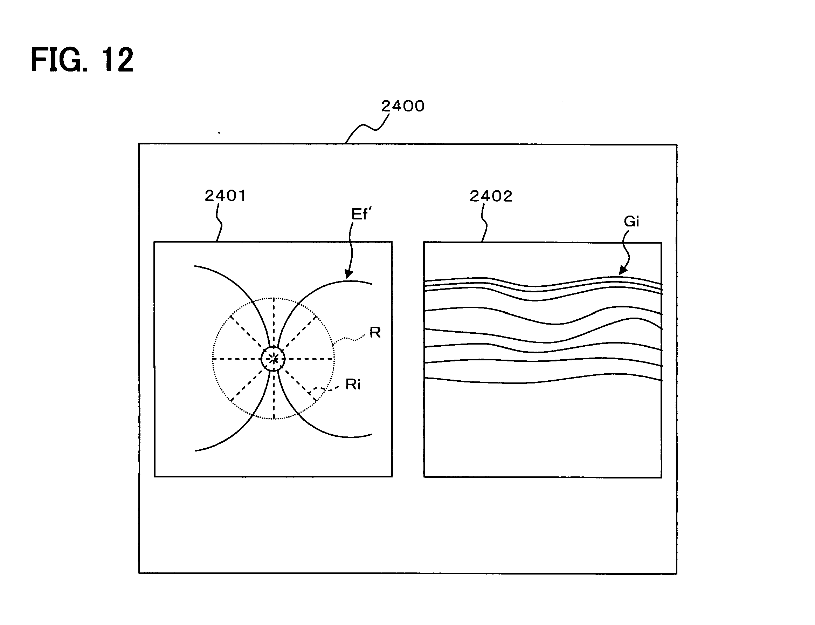

The controller 210 controls to display the acquired tomographic image in the OCT image display region 2402. Fig. 12 shows an example of a display mode of a tomographic image. On the fundus oculi image Ef', (the positions of) the scanning lines Ri are displayed. When the examiner designates one of the scanning lines Ri by the mouse 206 or the like, the controller 210 specifies a tomographic image based on the aforementioned positional information of the scanning lines or scanning points, and controls to display in the OCT image display region 2402. In Fig. 12, a tomographic image corresponding to the scanning line Ri is denoted by symbol Gi. When two or more scanning lines are designated, two or more tomographic images corresponding to these scanning lines may be displayed in the OCT image display region 2402.

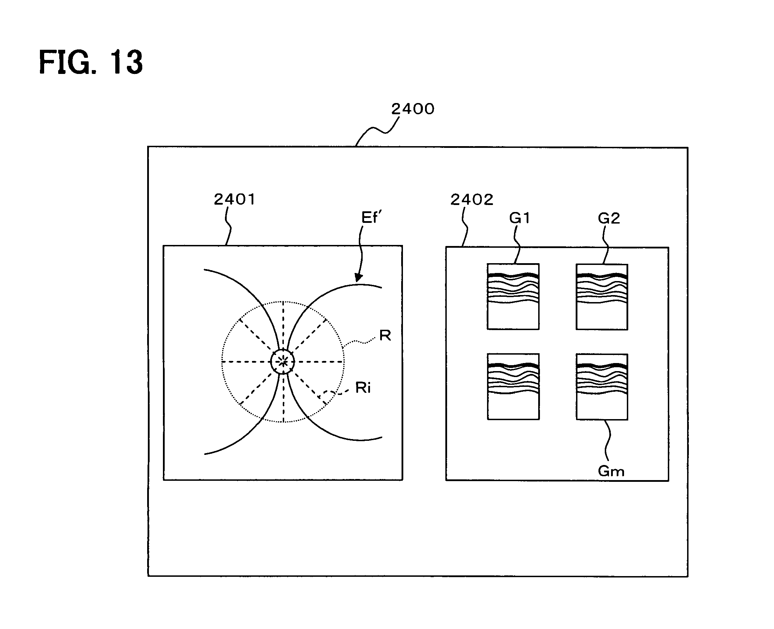

Further, Fig. 13 shows another example of a display mode for a tomographic image. In this display mode, a thumbnail image Gi (denoted by the same symbol) of the tomographic image Gi corresponding to each of the scanning lines Ri is displayed side by side in the OCT image display region 2402. When the examiner designates a desired thumbnail image, the controller 210 controls to zoom and display the tomographic image Gi corresponding to the designated thumbnail image Gi.

When the tomographic image Gi of the fundus oculi Ef is acquired, the 3-dimensional image forming part 231 of the image processor 230 firstly obtains the interval between the scanning lines Ri (S4). In a case where the scanning lines R1 to Rm are arranged at regular intervals, an angle θ between the adjacent scanning lines is θ = 180°/m. For example, the angle θ can be used as the interval between the scanning lines Ri.

Even if the scanning lines R1 to Rm are not arranged at regular intervals, it is possible to obtain an angle between the adjacent scanning lines, and use this angle as the interval between these adjacent scanning lines.

Further, as the interval between the scanning lines Ri, it is possible to use a distance between points that are equidistant from the center position C. This distance may be, for example, a linear distance defined by the x-y coordinate system, or a distance in the angular direction (length of arc) in the polar coordinate system with the origin at the center position C.

The 3-dimensional image forming part 231 obtains the interval between the adjacent scanning lines, for each point on each of the scanning lines Ri, as described above.

Then, the 3-dimensional image forming part 231 compares the intervals between the scanning lines Ri obtained in Step 4 with a threshold, and specifies a region in the scanning region R where the intervals between the scanning lines Ri are equal to or less than the threshold (S5). This process can be performed in the following manner, for example. This threshold is set to the upper limit value (or less) of the interval between scanning lines that allows the abovementioned interpolation process, and prestored in the hard disk drive 204 or the like.

First, on each of the scanning lines Ri, a portion where the interval between the adjacent scanning line are equal to or less than the threshold (referred to as a 'specific portion') is specified. Here, the scanning lines Ri are radially arranged, and the interval between adjacent scanning lines becomes smaller at points closer to the center position C. Therefore, the specific portion of each of the scanning lines Ri includes the center position C. Moreover, in this embodiment, each of the scanning lines Ri is linear, so that the specific portion of each of the scanning lines Ri is 'connected' in a mathematical sense.

Next, points that are the most distant from the center position C (namely, points at which the intervals between the adjacent scanning lines are the largest) in the specific portions of the respective scanning lines Ri are connected, and a region where the intervals between the scanning lines Ri are equal to or less than the threshold is determined. At the time of connection of points that are the most distant from the center position C, a polygonal region may be acquired by connecting adjacent points to each other with a straight line, or a closed-curve-shaped region may be acquired by connecting adjacent points to each other with a curved line. In the latter case, when the scanning lines Ri are arranged at regular intervals, the acquired closed curve becomes a circle.

When a region in the scanning region R is specified, the 3-dimensional image forming part 231 forms a 3-dimensional image of the fundus oculi Ef in this region based on partial images of the tomographic images Gi (S6).

The controller 210 controls to display the 3-dimensional image formed in Step 6 in response to, for example, an instruction by the examiner in the OCT image display region 2402 (S7).

### (Usage Pattern 2)

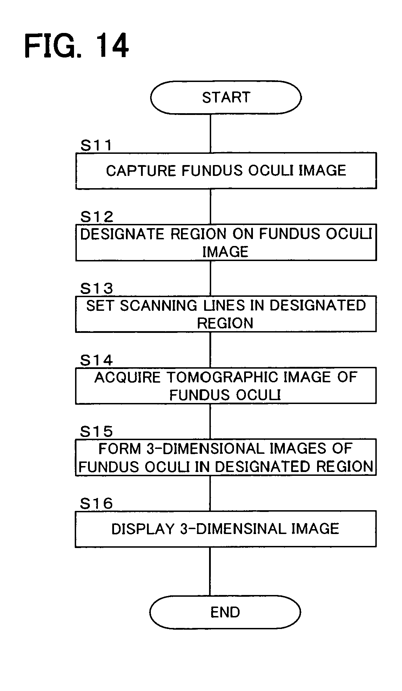

An example of a process of setting scanning lines depending on the size of a designated region and forming a 3-dimensional image will be described referring to a flowchart shown in Fig. 14.

First, the fundus oculi observation device 1 captures a fundus oculi image Ef' of the eye E (S11). The controller 210 controls the display 240A to display the captured fundus oculi image Ef' (see Fig.10).

Next, a region on the displayed fundus oculi image Ef' is designated (S12). Designation of the region is performed by, for example, an examiner operating the operation part 240B. This designated region is designated so as to include, for example, an image equivalent to an attention site such as optic papilla and a lesion in the fundus oculi Ef.

Then, the scanning line setting part 211 of the controller 210 sets the scanning lines Ri in this designated region based on the size of the region designated in Step 12 (S13). At this moment, the scanning line setting part 211 sets the scanning lines Ri of such a number that allows formation of a 3-dimensional image of the fundus oculi Ef in the designated region, as described above.

When the scanning lines Ri are set, the fundus oculi observation device 1 acquires a tomographic image of the fundus oculi Ef along each of the scanning lines Ri (S14).

Next, the 3-dimensional image forming part 231 forms a 3-dimensional image of the fundus oculi Ef in the region designated in Step 12, based on the tomographic images acquired in Step 14 (S 15).

The controller 210 controls to display the 3-dimensional image formed in Step 15, in response to, for example, an instruction of the examiner in the OCT image display region 2402 (S16).

### (Usage Pattern 3)

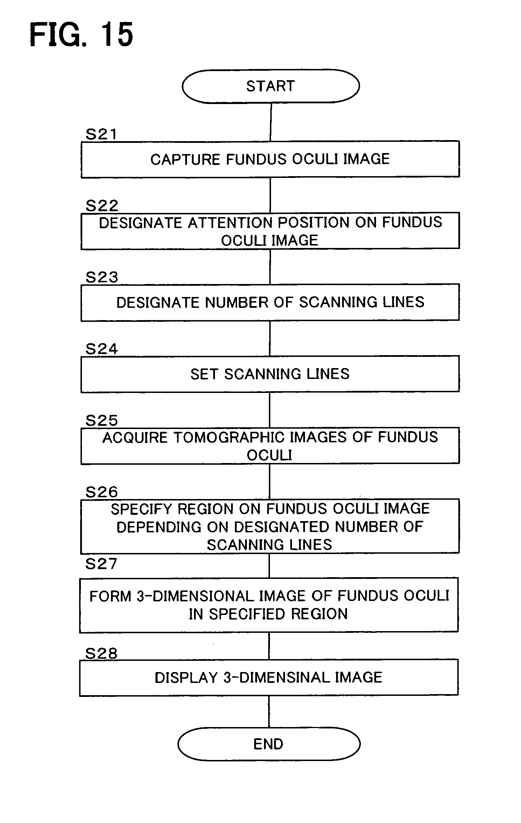

An example of a process of forming a 3-dimensional image of a region depending on a designated number of scanning lines will be described referring to a flowchart shown in Fig. 15.

First, the fundus oculi observation device 1 captures a fundus oculi image Ef' of the eye E (S21). The controller 210 controls the display 240A to display the captured fundus oculi image Ef' (see Fig.10).

The examiner designates an attention position on the displayed fundus oculi image Ef' (S22). As this attention position, for example, the position of the center of optic papilla, a lesion, or the like of the fundus oculi Ef is designated.

Next, the number of scanning lines is designated (S23). Designation of the number is performed by, for example, displaying a predetermined display screen and operating the operation part 240B.

Then, the scanning lines Ri are set on the fundus oculi Ef (fundus oculi image Ef') (S24). More specifically, the scanning line setting part 211 designates the scanning lines Ri by arranging the scanning lines Ri of the number designated in Step S23 in a radial pattern in which the attention position designated in Step S22 is the center position C. At this moment, the scanning lines Ri may be arranged at regular intervals, or may be arranged at irregular intervals. Moreover, such a configuration is available that it is possible to appropriately change the automatically set positions of the scanning lines Ri by operating the operation part 240B.

When the scanning lines Ri are set, the fundus oculi observation device 1 acquires a tomographic image of the fundus oculi Ef along each of the scanning lines Ri (S25).

Next, the 3-dimensional image forming part 231 specifies a region on the fundus oculi Ef (fundus oculi image Ef') depending on the number of scanning lines designated in Step 23 (S26). The region to be specified is a region in which the abovementioned interpolation process can be implemented with tomographic images along the designated number of scanning lines, namely, a region in which a 3-dimensional image can be formed with the designated number of scanning lines. This process in Step S26 can be implemented in the same way as in, for example, Step 4 and Step 5 of Usage Pattern 1.

Furthermore, the 3-dimensional image forming part 231 forms a 3-dimensional image of the fundus oculi Ef in the region specified in Step 26 (S27).

The controller 210 controls to display the 3-dimensional image formed in Step 27, in response to, for example, an instruction of the examiner (S28).

### [Advantageous Effects]

Actions and advantageous effects of the fundus oculi observation device 1 as described above will be described below.

The fundus oculi observation device 1 functions as an optical image measurement device capable of measuring an OCT image such as a tomographic image of the fundus oculi Ef. This fundus oculi observation device 1 is configured so as to scan a target position of a signal light LS along a plurality of scanning lines Ri formed radially, form a tomographic image Gi of each scanning line Ri, and form a 3-dimensional image of the fundus oculi Ef based on the plurality of tomographic images Gi.

Since the fundus oculi observation device 1 as described above is configured so as to scan with the signal light LS along the scanning lines Ri arranged radially, it can effectively fix the eye E. That is, with conventional scan, a subject tends to follow a linear image moving in the vertical direction with eyes and move the viewpoint in the vertical direction, whereas since this fundus oculi observation device 1 enables the subject to view a linear image rotating about the center position C, the eyes are less likely to follow the image compared to the case of the conventional scan. This makes it possible to effectively fix the eye E. In addition, because the eye E can be effectively fixed, it becomes possible to acquire a 3-dimensional image of higher quality than before.

Further, according to the fundus oculi observation device 1, it is possible to acquire a high-quality image while shortening a measurement time, for the following reasons.

In a conventional optical image measurement device, a plurality of scanning lines extending in the horizontal direction are arranged in the vertical direction, so that the intervals between the adjacent scanning lines are equal (in particular, the intervals between the scanning lines are equal throughout the scanning region R). Further, in an object region for acquiring a 3-dimensional image, the intervals between the scanning lines need to be set small. In particular, in a conventional configuration, even when it is desired to acquire a 3-dimensional image only for a partial region within the scanning region R, it is necessary to set the intervals between the scanning lines small throughout the scanning region R. Therefore, in the conventional configuration, it is required to scan with a signal light along a number of scanning lines, resulting in a longer measurement time.

Meanwhile, according to the fundus oculi observation device 1 related to this embodiment, the scanning lines Ri arranged radially are used, so that the intervals between the scanning lines vary from position to position. To be specific, the intervals between the scanning lines are small at positions close to the center position C, and the intervals between the scanning lines are large at positions distant from the center position C. Therefore, when a region near the center position C is an object region for acquiring a 3-dimensional image, it is possible to acquire a high-quality 3-dimensional image without increasing the number of scanning lines specifically. In other words, according to this fundus oculi observation device 1, it is possible to acquire a high-quality 3-dimensional image while shortening the time for scanning with the signal light LS.

In particular, it is possible to acquire a high-quality 3-dimensional image while shortening a measurement time, by forming a 3-dimensional image in a region including an intersection position (center position C) of the scanning lines Ri, or a 3-dimensional image in a region where intervals between scanning lines are equal to or less than a threshold.

In addition, the fundus oculi observation device 1 is configured so as to, when a region on the fundus oculi Ef (fundus oculi image Ef') is designated, radially set in the region the scanning lines Ri of a number depending on the size of this designated region, form a tomographic image Gi on each scanning line Ri, and form a 3-dimensional image of the fundus oculi Ef in the designated region based on the tomographic images Gi.

Consequently, it is possible to measure only the scanning lines of a number required to acquire a 3-dimensional image in the designated region, so that it becomes possible to acquire a high-quality 3-dimensional image while shortening the measurement time.

Further, since the fundus oculi observation device 1 is configured so as to, when the number of scanning lines is designated, radially set the designated number of scanning lines Ri on the fundus oculi Ef (fundus oculi image Ef'), the examiner can set a desired number depending on the size or the like of a site to be observed.

Furthermore, the fundus oculi observation device 1 is configured so as to form a tomographic image Gi on each of the designated number of scanning lines and form a 3-dimensional image of the fundus oculi Ef in a region depending on the designated number based on the tomographic image Gi. At this moment, the center position C is designated on an attention position of the fundus oculi Ef as described above. Therefore, by setting relatively small number of scanning lines, it is possible to quickly acquire a 3-dimensional image near the attention position. In addition, by considering the number of scanning lines, it is possible to acquire a high-quality image while shortening the measurement time for acquiring a 3-dimensional image.

### [Modification]

The configuration described above is merely a specific example for favorably implementing the present invention. Therefore, it is possible to properly make any modification within the scope and intent of the present invention.

For example, in the embodiment described above, the difference between the light path of a signal light and the light path of a reference light (difference in optical path length) is changed by changing the position of the reference mirror 174, but the method for changing the difference in optical path length is not limited to this. For instance, it is possible to change the difference in optical path length by integrally moving the retinal camera unit 1A and the OCT unit 150 with respect to the eye to be examined and changing the optical path length of the signal light LS. Furthermore, it is also possible to change the difference in optical path length by moving a measurement object in a depth direction (z-direction).

The fundus oculi observation device described in the above embodiment comprises an optical image measurement device of Fourier domain type, but it is also possible to apply the configuration of the present invention to an optical image measurement device of Time Domain type. The time domain type of optical image measurement device is described in, for example, Japanese Unexamined Patent Application Publication 2005-241464. Moreover, it is also possible to apply the configuration of the present invention to an optical image measurement device of any other type such as a Swept Source type.

In the aforementioned embodiment, a device for obtaining an OCT image of the fundus has been explained. However, the composition of the abovementioned embodiment can be applied to a device that is capable of obtaining an OCT image of other sites in the subject eyes, such as the cornea. In addition, the composition of the abovementioned embodiment can be also applied to a device that is capable of obtaining an OCT image of any measurement object (subject in other medical, biological, and industrial fields), for which an image can be obtained by OCT technology.

The modifications described above can also be appropriately applied to a second embodiment described below.

### <Second Embodiment>

A second embodiment of the optical image measurement device according to the present invention will be described. This embodiment shows generation of predetermined characteristic information based on a plurality of tomographic images of a measurement object. In this embodiment, description is made for a case of generating information representing the distribution of thickness of layers composing a fundus oculi, as the characteristic information.

The retina of a fundus oculi is composed of an inner limiting membrane, a nerve fiber layer, a ganglion cell layer, a plexiform layer, an inner nuclear layer, an outer plexiform layer, an outer nuclear layer, an outer limiting membrane, a photoreceptor layer, a retinal pigment epithelium layer, and so on. In addition, a choroid and a sclera are present under the retina.

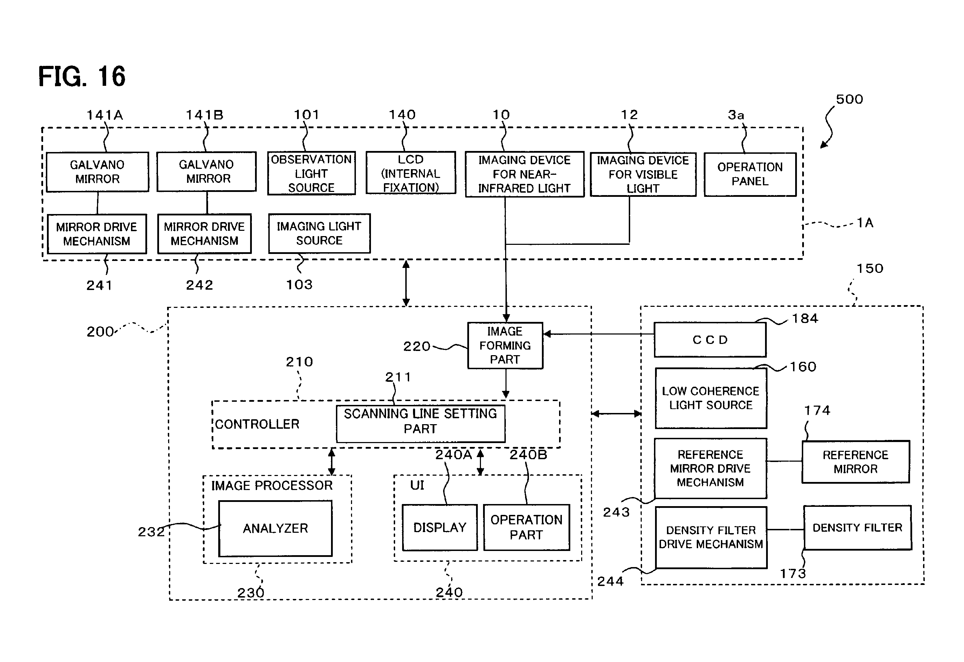

As shown in Fig. 16, a fundus oculi observation device 500 (optical image measurement device) according to this embodiment has the same optical system and hardware configuration as those of the fundus oculi observation device 1 according to the first embodiment (see Fig. 1 through Fig. 4). Hereinafter, the same components as those in the first embodiment will be denoted by the same symbols. In addition, also in this embodiment, the scanning lines Ri of the signal light LS are radially arranged, as in the first embodiment (see Fig. 7 and Fig. 8). Furthermore, various types of processes described below are executed based on the control program 204a shown in Fig. 4.

Fig. 16 shows a configuration of a control system of the fundus oculi observation device 500. The control system of the fundus oculi observation device 500 is configured almost in the same manner as in the first embodiment (see Fig. 5). In this regard, however, the image processor 230 of the fundus oculi observation device 500 does not require the 3-dimensional image forming part 231. Moreover, the image processor 230 in this embodiment is provided with an analyzer 232.

The analyzer 232 generates characteristic information of the fundus oculi Ef by analyzing the tomographic image Gi formed by the image forming part 220, and is equivalent to an example of the "analyzer" of the present invention. The analyzer 232 executes, for example, (1) a layer-position analysis process, (2) a fundus oculi thickness calculation process, and (3) a characteristic-information generation process, as described below.

### (Layer-position analysis process)

The analyzer 232 executes a process for forming a 3-dimensional image of the fundus oculi Ef as in the 3-dimensional image forming part 231 in the first embodiment and for obtaining a position of a predetermined layer of the fundus oculi Ef in this 3-dimensional image. More specifically, the analyzer 232 obtains the layer position of the fundus oculi Ef, for example, in the following.

The analyzer 232 first executes a preliminary process for making it easier to obtain the layer position of the fundus oculi Ef in the 3-dimensional image. As this preliminary process, for example, any image processing, such as tone conversion, image enhancement, threshold processing, contrast conversion, binarization, edge detection, image averaging, image smoothing, and filtering, can appropriately be executed. It is also possible to execute image processing by combining these appropriately.

Next, the analyzer 232 analyzes the pixel value (e.g., luminance value) of pixels that constitute the preprocessed 3-dimensional image line by line along the depth-wise direction (z-direction) of the fundus oculi Ef.

In other words, because the 3-dimensional image to be analyzed is composed of a plurality of images in the depth-wise direction that are 2-dimensionally arranged in the x-y direction, the analyzer 232 specifies a pixel equivalent to the border between adjacent layers by referencing the pixel value of pixels that constitute each image in the depth-wise direction in order of precedence along the depth-wise direction.

At this moment, it is possible to specify a pixel equivalent to the bordering position between the layers by using a filter having spread only in the depth-wise direction (e.g., differential filter). Alternatively, it is possible to specify the layer position in an optional way, such as detecting the edge of a pixel by utilizing a filter that extends in two directions-depth-wise direction and direction perpendicular-thereto (area filter).

Furthermore, the analyzer 232 obtains an image region equivalent to the bordering position between the layers of the fundus oculi Ef in a 3-dimensional image, and also obtains an image region equivalent to the layer of the fundus oculi Ef. In other words, the fundus oculi Ef is composed of a plurality of layers, so that specifying a layer becomes synonymous with specifying the bordering position between the layers.

The analyzer 232 obtains the layer position of at least any one of the abovementioned various layers of the fundus oculi Ef (the bordering position between the layers) through the process as described.

### (Fundus oculi thickness calculation process)

The analyzer 232 performs a process for calculating the thickness of a specific site of the fundus oculi Ef based on the position of the layer of the fundus oculi Ef obtained by the abovementioned layer-position analysis process. Here, a specific site of the fundus oculi Ef refers to one or more layers of the abovementioned plurality of layers of the fundus oculi Ef. For example, the retinal pigment epithelium layer alone is equivalent to a 'sdpecific site,' and a plurality of layers from the inner limiting membrane to the inner nuclear layer is also equivalent to a 'specific site.'

Further, as the 'specific site' for which the thickness is calculated, there are in particular the thickness from the inner limiting membrane to the nerve fiber layer (nerve fiber layer thickness), thickness from the inner limiting membrane to the inner nuclear layer (photoreceptor inner and outer segment) (retinal thickness), thickness from the inner limiting membrane to the retinal pigment epithelium layer (retinal thickness), and the like. The second and third examples of these three examples each represent retinal thickness, but a plurality of definitions are typically used appropriately for retinal thickness.

An example of a fundus oculi thickness calculation process is described. The analyzer 232 obtains a bordering position between layers of the fundus oculi Ef in a 3-dimensional image, as described above. At this moment, the analyzer 232 obtains at least two bordering positions and calculates a distance between predetermined two bordering positions.

More specifically, the analyzer 232 calculates, for images in each depth-wise direction that constitutes a 3-dimensional image, the distance between pixels that are equivalent to each of the predetermined two bordering positions (distance in the depth-wise direction). At this moment, a coordinate value is assigned according to the abovementioned x-y-z coordinate system for each pixel of an image in the depth-wise direction (x coordinate values and y coordinate values of pixels in an image in each depth-wise direction are constant). The analyzer 232 can calculate a distance in the z-direction between pixels from this coordinate value. In addition, the analyzer 232 can also calculate a target distance based on the number of pixels between the pixels equivalent to each of the two bordering positions and the distance between adjacent pixels (known).

### (Characteristic information generation process)

The analyzer 232 performs a process of generating distribution information that represents distribution of thickness of a predetermined position of the fundus oculi Ef based on the result of calculation by the fundus oculi thickness calculation process. As this distribution information, it is possible to generate graphic information that represents 1-dimensional distribution of thickness of a layer at an optional cross-sectional position (referred to as layer-thickness distribution graph), image information that represents 2-dimensional distribution of thickness of a layer in the x-y direction (surface direction of the fundus oculi Ef) (referred to as layer thickness distribution image), and the like. Here, the thickness of a layer of the fundus oculi Ef corresponds to an example of the "characteristic value" of the present invention.

Here, the layer-thickness distribution graph is a graph that depicts the thickness of a layer at each cross-sectional position by, for example, showing the cross-sectional positions on the horizontal axis and the thickness of layers on the vertical axis. Here, it is also possible to perform statistical operation, such as dividing a targeted cross-sectional position into a plurality of partial regions and obtaining an average value of thickness of layers for each partial region, to generate a layer thickness distribution graph that displays the result of this statistical operation for each partial region. In this case, it is desirable to determine the size of each partial region depending on an interval between scanning lines Ri. In other words, in a site where an interval between the scanning lines Ri is small, an interpolation process for forming a 3-dimensional image can be performed with relatively high precision, so that it is possible to set a partial region at that site to a small size. Meanwhile, in a site where an interval between the scanning lines Ri is large, the precision of an interpolation process for forming a 3-dimensional image is relatively low, so that a partial region at that site is set to a large size. By thus setting the sizes of the partial regions, it is possible to present the thickness of a layer in detail for a portion with high precision of a 3-dimensional image, and present roughly for a portion with low precision. Since the scanning lines Ri are radially arranged also in this embodiment, the size of the partial region at a site close to the center position C is set to small, and the size of the partial region at a site distant from the center position C is set to large.

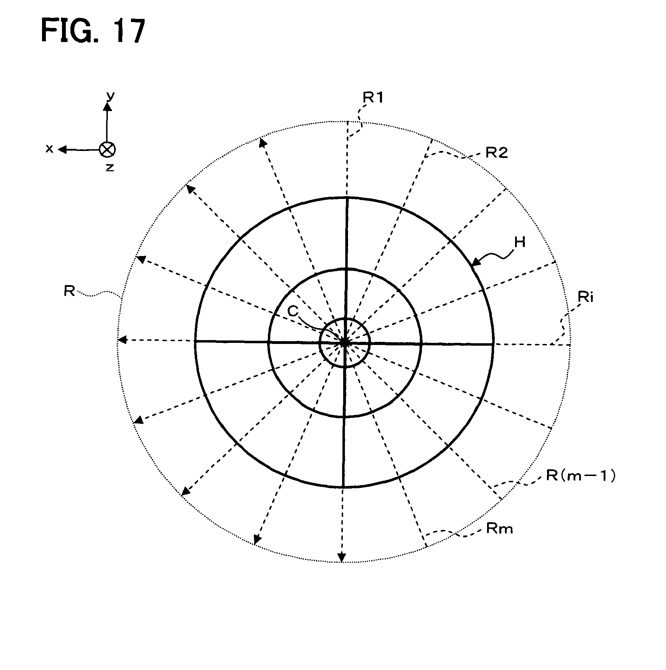

Further, the layer-thickness distribution image expresses the distribution of thickness of layers in the x-y direction in different display colors or by shading of display colors. Fig. 17 shows an example of the layer-thickness distribution image. A layer-thickness distribution image H shown in Fig. 17 has three concentric borderlines about the center position C of the scanning lines Ri and borderlines in the x-direction and y-direction, and divides a region in the scanning region R (e.g., a region where a 3-dimensional image can be formed) into twelve partial regions. As in the abovementioned layer-thickness distribution graph, the size of a partial region at a site close to the center position C is set smaller than the size of a partial region at a site distant from the center position C. In other words, the size of a partial region at a site where an interval between the scanning lines Ri is small is set smaller than the size of a partial region at a site where an interval between the scanning lines Ri is large. Furthermore, although illustrations are omitted, each partial region is displayed in a display color or the like depending on the calculation result (such as an average value) of the statistical operation of thickness of layers in the region.

The fundus oculi observation device 500 that operates in the above manner has actions and advantageous effects as described below.

The fundus oculi observation device 500 divides the low-coherence light L0 emitted from the low-coherence light source 160 into the signal light LS and the reference light LR, superimposes the signal light LS passed through the fundus oculi Ef on the reference light LR to generate the interference light LC, and detects it with the CCD 184. The controller 210 controls the scanning unit 141 to scan a target position of the signal light LS along a plurality of radially-arranged scanning lines Ri. The image-forming part 220 forms a tomographic image Gi on each scanning line Ri based on the result detected by the CCD 184. The analyzer 232 analyzes the tomographic image Gi, and generates distribution information (characteristic information) such as a layer-thickness distribution graph and a layer-thickness distribution image of the fundus oculi Ef.

Since this fundus oculi observation device 500 scans with the signal light LS along the scanning lines Ri radially arranged, it is possible to effectively fix the eye E, and it is possible to acquire characteristic information with high precision.

Further, according to the fundus oculi observation device 500, it is possible to shorten a measurement time, and also acquire a high-quality image and high-precision characteristic information.

A modification of the optical image measurement device according to this embodiment will be described. The aforementioned fundus oculi observation device 500 acquires the distribution of thickness of layers of the fundus oculi Ef as characteristic information, but characteristic information is not limited to this. For example, it is possible to configure so as to obtain characteristic information that represents the distribution of the blood flow velocity in blood vessels of the fundus oculi Ef or characteristic information that represents the distribution of the presence or absence, number of segments, or the like, of a lesion. In addition, it is possible to configure so as to acquire, also for a measurement object other than the fundus oculi, characteristic information that represents any characteristic that can be determined from an OCT image of the measurement object.

The optical image measurement device according to the present invention is configured to scan a target position of a signal light emitted to a measurement object along a plurality of radially-arranged scanning lines, form tomographic images at the respective scanning lines based on the result detected by the detector, and form a 3-dimensional image of the measurement object based on these tomographic images.

By scanning a target position of a signal light along radially-arranged scanning lines as described above, it is possible to fix an eye more effectively than by scanning the target position of the signal light in the horizontal and vertical directions as conventional. In other words, in the conventional scanning mode, a subject views a linear image moving in the vertical direction and, in accordance with the movement of this image, the viewpoint is guided in the vertical direction, so that there is a case where fixation cannot be effectively done. However, in scan with a signal light along the radially-arranged scanning lines as in the present invention, a subject views a linear image rotating about a crossing position of the scanning lines, so that it is possible to more effectively fix the eye, as compared with in the conventional scanning mode.

Further, in the conventional configuration, a plurality of scanning lines extending in the horizontal direction are arranged in the vertical direction, and therefore, the intervals between the adjacent scanning lines are equal. Furthermore, in an object region for acquiring a 3-dimensional image, the intervals between the scanning lines need to be set small in order to favorably perform an interpolation process and so on. Therefore, with the conventional configuration, even when it is desired to acquire a 3-dimensional image only for a partial region in the scanning region, the intervals between the scanning lines must be set small throughout the scanning region, and thus, a number of scanning lines need to be set, which results in a longer measurement time. Meanwhile, radially-arranged scanning lines are used in the configuration related to the present invention, so that the intervals between the scanning lines vary from position to position. To be specific, the intervals between the scanning lines are small at sites close to the crossing position of the scanning lines, and the intervals between the scanning lines are large at sites distant from the crossing position. Therefore, according to the present invention, by placing an object region for acquiring a 3-dimensional image in proximity to the crossing position, it is possible to acquire a high-quality 3-dimensional image without increasing the number of scanning lines. In other words, according to the present invention, it is possible to acquire a high-quality 3-dimensional image while shortening a measurement time by shortening a scanning time with a signal light.

Further, the optical image measurement device according to the present invention is capable of scanning a target position of a signal light along a plurality of radially-arranged scanning lines, forming tomographic images for the respective scanning lines based on the result detected by the detector, and analyzing these tomographic images to generate characteristic information of a measurement object. This characteristic information is distribution information representing distribution of characteristic values of a measurement object, and the like.

According to the present invention as described above, by radially scanning with a signal light, it is possible to effectively fix an eye. Moreover, similarly to the above, it is possible to acquire a high-quality image while shortening a measurement time. Besides, since it is possible to acquire a high-quality image in this manner, it is possible to acquire the characteristic information with high precision.

## Claims

1. An optical image measurement device that has:
a light source configured to emit a low-coherence light;
an interference-light generator configured to generate an interference light by dividing the emitted low-coherence light into a signal light and a reference light and superimposing the signal light having passed through a measurement object on the reference light;
a scanner configured to scan a target position of the signal light on the measurement object; and
a detector configured to detect the generated interference light,
and that forms an image of the measurement object based on a result detected by the detector,
the optical image measurement device comprising:
a controller configured to control the scanner to scan the target position along a plurality of radially-arranged scanning lines; and
an image forming part configured to form a tomographic image on each of the plurality of scanning lines based on the result detected by the detector, and form a 3-dimensional image of the measurement object based on the plurality of tomographic images having been formed.

2. The optical image measurement device according to Claim 1, wherein:
the plurality of scanning lines are radially arranged in a scanning region on the measurement object; and
the image forming part forms a 3-dimensional image of the measurement object in a region in the scanning region including an intersection position of the plurality of scanning lines.

3. The optical image measurement device according to Claim 1, wherein:
the plurality of scanning lines are radially arranged in a scanning region on the measurement object; and
the image forming part forms a 3-dimensional image of the measurement object in a region in the scanning region in which an interval between the scanning lines is equal to or less than a threshold.

4. The optical image measurement device according to Claim 1, further comprising:
a designating part configured to designate a region on the measurement object, wherein:
the controller controls the scanner to scan the target position of the signal light along radially-arranged scanning lines of a number determined by a size of the designated region; and
the image forming part forms a tomographic image on each of the number of radially-arranged scanning lines, and forms a 3-dimensional image of the measurement object in the designated region based on the formed tomographic image.

5. The optical image measurement device according to Claim 1, further comprising:
an operation part configured to designate a number of scanning lines, wherein:
the controller controls the scanner to scan the target position of the signal light along the designated number of radially-arranged scanning lines.

6. The optical image measurement device according to Claim 5, wherein:
the image forming part forms a tomographic image on each of the radially-arranged scanning lines of the number designated by the operation part, and forms a 3-dimensional image of the measurement object in a region depending on the designated number, based on the formed tomographic image.

7. An optical image measurement device that has:
a light source configured to emit a low-coherence light;
an interference-light generator configured to generate an interference light by dividing the emitted low-coherence light into a signal light and a reference light and superimposing the signal light having passed through a measurement object on the reference light;
a scanner configured to scan a target position of the signal light on the measurement object; and
a detector configured to detect the generated interference light,
and that forms an image of the measurement object based on a result detected by the detector,
the optical image measurement device comprising:
a controller configured to control the scanner to scan the target position along a plurality of radially-arranged scanning lines;
an image forming part configured to form a tomographic image on each of the plurality of scanning lines based on the result detected by the detector; and
an analyzer configured to analyze the plurality of formed tomographic images to generate characteristic information representing characteristics of the measurement object.

8. The optical image measurement device according to Claim 7, wherein:
the plurality of scanning lines are radially arranged in a scanning region on the measurement object; and
the analyzer generates, as the characteristic information, distribution information representing distribution of characteristic values in the scanning region.

9. The optical image measurement device according to Claim 8, wherein:
the analyzer divides the scanning region into two or more partial regions depending on an interval between the scanning lines and
generates the distribution information by allocating the characteristic values to each of the two or more partial regions.

10. The optical image measurement device according to Claim 8, wherein:
the measurement object has a layered structure; and
the analyzer generates, as distribution of the characteristic values, the distribution information representing the distribution of thickness of layers of the measurement object.
